# EUROPEAN PATENT APPLICATION

(11) **EP 4 621 056 A1**
(43) Date of publication of application: **24.09.2025**
(21) Application number: 23891502.9
(22) Date of filing: 13.11.2023
(51) Int. Cl.: C12N 15/12, A61K 35/12, A61K 35/35, A61P 7/04, C07K 14/755, C12N 5/10, C12N 5/077, C12N 15/63, C12N 15/867

(54) **CELLS FOR EX-VIVO GENE THERAPY THAT PRODUCE MODIFIED BLOOD COAGULATION FACTOR VIII**

(30) Priority: 14.11.2022 JP 2022182143
(71) Applicant: Cellgentech, Inc., Chiba-shi, Chiba 260-0856 (JP)
(72) Inventor: ONITAKE Akinobu, Chiba-shi, Chiba 260-0856 (JP); AOYAGI Yasuyuki, Chiba-shi, Chiba 260-0856 (JP); MATSUURA Yuta, Chiba-shi, Chiba 260-0856 (JP); KAYABA Atsuko, Chiba-shi, Chiba 260-0856 (JP); YASUNAGA Kunio, Chiba-shi, Chiba 260-0856 (JP); YAMAMOTO Tokuo, Chiba-shi, Chiba 260-0856 (JP); TANIO Masami, Chiba-shi, Chiba 260-0856 (JP); ASO Masayuki, Chiba-shi, Chiba 260-0856 (JP)
(74) Representative: Louis Pöhlau Lohrentz
(86) International application number: PCT/JP2023/040665
(87) International publication number: WO 2024/106355

(57) **Abstract**

An object of the present invention is to provide a novel cell for *ex vivo* gene therapy which produces a FVIII variant. The cell for *ex vivo* gene therapy of the present invention as a solution thereof produces a variant of human FVIII having the amino acid sequence at positions 20 to 2351 of the amino acid sequence of SEQ ID NO: 1 in which the B domain (the amino acid sequence at positions 760 to 1667) has been modified, thereby, the number of Asn residues subjected to N-linked glycosylation is four to six from the N-terminus and the function of the furin recognition site is lacking.

## Description

### Technical Field

The present invention relates to a cell for *ex vivo* gene therapy which produces a blood coagulation factor VIII variant.

### Background Art

Blood coagulation factor VIII ("FVIII" below) is one of factors responsible for hemostatic mechanism, and congenital abnormality of FVIII gene leads to lack of or a decrease in the blood FVIII concentration and causes a hemostatic disorder called hemophilia A. To treat hemophilia A, plasma-derived FVIII preparations have been long used, but recombinant preparations are also clinically applied today. Human FVIII is a glycoprotein composed of 2332 amino acid residues. A precursor (the amino acid sequence is shown in SEQ ID NO: 1) composed of 2351 amino acid residues including a signal peptide composed of 19 amino acid residues is expressed mainly in the cells of the liver, and then a full-length form composed of 2332 amino acid residues is formed through cleavage of the signal peptide, glycosylation and the like. The full-length form has a domain structure A1-A2-B-A3-C1-C2, and linker regions called a1, a2 and a3 are located between the A1 domain and the A2 domain, between the A2 domain and the B domain and between the B domain and the A3 domain, respectively. The full-length form turns into a mature form by conversion into a double chain having a heavy chain and a light chain through cleavage between Arg1648 at the C-terminus of the B domain and Glu1649 at the N-terminus of the a3 linker region (position 1667 and position 1668 of the amino acid sequence of SEQ ID NO: 1) by furin in the Golgi apparatus, and the mature form is secreted from the cells into the circulating blood and then converted into an active form through cleavage between Arg372 at the C-terminus of the a1 linker region and Ser373 at the N-terminus of the A2 domain, between Arg740 at the C-terminus of the a2 linker region and Ser741 at the N-terminus of the B domain and between Arg1689 at the C-terminus of the a3 linker region and Ser1690 at the N-terminus of the A3 domain by thrombin to exhibit certain functions.

Hemophilia A is a hereditary disorder caused by a single gene and thus is believed to be a target disease suitable for gene therapy, and therefore gene therapy for hemophilia A has been recently researched and developed actively (for example, Non-Patent Document 1). Gene therapy is roughly classified by the methods into *in vivo* gene therapy, in which a vector for expressing a target gene *in vivo* is directly administered to a patient, and *ex vivo* gene therapy, in which cells are removed from a patient and, after introducing a target gene, returned to the patient to express the target gene *in vivo.*

Regarding the research and the development of *ex vivo* gene therapy for hemophilia A, for example, an attempt has been made to introduce a polynucleotide for producing a FVIII variant which lacks the B domain of human FVIII *in vivo* into cells removed from a patient using a lentiviral vector, which is highly safe and can express a gene stably for a long time because the target gene is efficiently inserted into the host cell genome, and then return the cells to the patient (ID: NCT03818763 introduced in Non-Patent Document 1). The research and the development of ex *vivo* gene therapy for hemophilia A, however, is considered to be still in progress.

### Prior Art Documents

### Non-Patent Document

Non-Patent Document 1: Yuji Kashiwakura et al., Advances in gene therapy for hemophilia, Japanese Journal of Thrombosis and Hemostasis, 2021;32(1):17-25

### Summary of the Invention

### Problems that the Invention is to Solve

An object of the present invention is to provide a novel cell for *ex vivo* gene therapy which produces a FVIII variant.

### Means for Solving the Problems

The cell for *ex vivo* gene therapy of the present invention produces a variant of human FVIII having the amino acid sequence at positions 20 to 2351 of the amino acid sequence of SEQ ID NO: 1 in which the B domain (the amino acid sequence at positions 760 to 1667) has been modified, thereby, the number of Asn residues subjected to N-linked glycosylation is four to six from the N-terminus and the function of the furin recognition site is lacking.

Moreover, in the cell for *ex vivo* gene therapy of the present invention, the FVIII variant is any of (a) to (c) below.
(a) a protein having the amino acid sequence at positions 20 to 1607 of the amino acid sequence of SEQ ID NO: 2
(b) a protein which has an amino acid sequence containing deletion, substitution, addition or insertion of one or a plurality of amino acid residues in the amino acid sequence at positions 20 to 1607 of the amino acid sequence of SEQ ID NO: 2 and which has an equivalent biological activity to that of the protein in (a)
(c) a protein which has an amino acid sequence having a sequence identity of at least 90% with the amino acid sequence at positions 20 to 1607 of the amino acid sequence of SEQ ID NO: 2 and which has an equivalent biological activity to that of the protein in (a)

Moreover, in the cell for *ex vivo* gene therapy of the present invention, the FVIII variant is any of (a) to (c) below.
(a) a protein having the amino acid sequence at positions 20 to 1652 of the amino acid sequence of SEQ ID NO: 3
(b) a protein which has an amino acid sequence containing deletion, substitution, addition or insertion of one or a plurality of amino acid residues in the amino acid sequence at positions 20 to 1652 of the amino acid sequence of SEQ ID NO: 3 and which has an equivalent biological activity to that of the protein in (a)
(c) a protein which has an amino acid sequence having a sequence identity of at least 90% with the amino acid sequence at positions 20 to 1652 of the amino acid sequence of SEQ ID NO: 3 and which has an equivalent biological activity to that of the protein in (a)

Moreover, in the cell for *ex vivo* gene therapy of the present invention, the FVIII variant is any of (a) to (c) below.
(a) a protein having the amino acid sequence at positions 20 to 1670 of the amino acid sequence of SEQ ID NO: 4
(b) a protein which has an amino acid sequence containing deletion, substitution, addition or insertion of one or a plurality of amino acid residues in the amino acid sequence at positions 20 to 1670 of the amino acid sequence of SEQ ID NO: 4 and which has an equivalent biological activity to that of the protein in (a)
(c) a protein which has an amino acid sequence having a sequence identity of at least 90% with the amino acid sequence at positions 20 to 1670 of the amino acid sequence of SEQ ID NO: 4 and which has an equivalent biological activity to that of the protein in (a)

Moreover, in the cell for *ex vivo* gene therapy of the present invention, the Pro residue at position 758 of the amino acid sequence of SEQ ID NO: 1 has been substituted with another amino acid residue in the FVIII variant.

Moreover, in the cell for *ex vivo* gene therapy of the present invention, the other amino acid residue is a polar uncharged amino acid residue selected from Thr residue, Ser residue, Asn residue and Gln residue.

Moreover, in the cell for *ex vivo* gene therapy of the present invention, the FVIII variant is any of (a) to (c) below.
(a) a protein having the amino acid sequence at positions 20 to 1652 of the amino acid sequence of SEQ ID NO: 5
(b) a protein which has an amino acid sequence containing deletion, substitution, addition or insertion of one or a plurality of amino acid residues in the amino acid sequence at positions 20 to 1652 of the amino acid sequence of SEQ ID NO: 5 and which has an equivalent biological activity to that of the protein in (a)
(c) a protein which has an amino acid sequence having a sequence identity of at least 90% with the amino acid sequence at positions 20 to 1652 of the amino acid sequence of SEQ ID NO: 5 and which has an equivalent biological activity to that of the protein in (a)

Moreover, in the cell for *ex vivo* gene therapy of the present invention, the FVIII variant is any of (a) to (c) below.
(a) a protein having the amino acid sequence at positions 20 to 1652 of the amino acid sequence of SEQ ID NO: 14
(b) a protein which has an amino acid sequence containing deletion, substitution, addition or insertion of one or a plurality of amino acid residues in the amino acid sequence at positions 20 to 1652 of the amino acid sequence of SEQ ID NO: 14 and which has an equivalent biological activity to that of the protein in (a)
(c) a protein which has an amino acid sequence having a sequence identity of at least 90% with the amino acid sequence at positions 20 to 1652 of the amino acid sequence of SEQ ID NO: 14 and which has an equivalent biological activity to that of the protein in (a)

Moreover, in the cell for *ex vivo* gene therapy of the present invention, the FVIII variant is any of (a) to (c) below.
(a) a protein having the amino acid sequence at positions 20 to 1652 of the amino acid sequence of SEQ ID NO: 15
(b) a protein which has an amino acid sequence containing deletion, substitution, addition or insertion of one or a plurality of amino acid residues in the amino acid sequence at positions 20 to 1652 of the amino acid sequence of SEQ ID NO: 15 and which has an equivalent biological activity to that of the protein in (a)
(c) a protein which has an amino acid sequence having a sequence identity of at least 90% with the amino acid sequence at positions 20 to 1652 of the amino acid sequence of SEQ ID NO: 15 and which has an equivalent biological activity to that of the protein in (a)

Moreover, in the cell for *ex vivo* gene therapy of the present invention, the FVIII variant is any of (a) to (c) below.
(a) a protein having the amino acid sequence at positions 20 to 1652 of the amino acid sequence of SEQ ID NO: 16
(b) a protein which has an amino acid sequence containing deletion, substitution, addition or insertion of one or a plurality of amino acid residues in the amino acid sequence at positions 20 to 1652 of the amino acid sequence of SEQ ID NO: 16 and which has an equivalent biological activity to that of the protein in (a)
(c) a protein which has an amino acid sequence having a sequence identity of at least 90% with the amino acid sequence at positions 20 to 1652 of the amino acid sequence of SEQ ID NO: 16 and which has an equivalent biological activity to that of the protein in (a)

Moreover, in the cell for *ex vivo* gene therapy of the present invention, the cell is an adipocyte.

Moreover, the composition for transplantation of the present invention contains the cell for *ex vivo* gene therapy of the present invention and a pharmaceutically acceptable carrier.

Moreover, the composition for transplantation of the present invention further contains an extracellular matrix component.

Moreover, the composition for transplantation of the present invention further contains an angiogenic factor.

Moreover, the method for producing a cell for *ex vivo* gene therapy of the present invention includes introducing an expression vector having a polynucleotide that encodes at least an amino acid sequence of a variant of human FVIII having the amino acid sequence at positions 20 to 2351 of the amino acid sequence of SEQ ID NO: 1 in which the B domain (the amino acid sequence at positions 760 to 1667) has been modified, thereby, the number of Asn residues subjected to N-linked glycosylation is four to six from the N-terminus and the function of the furin recognition site is lacking into a cell which can be transplanted into the body of a subject of the *ex vivo* gene therapy.

Moreover, in the method for producing a cell for ex *vivo* gene therapy of the present invention, the expression vector is a viral vector.

Moreover, in the method for producing a cell for ex *vivo* gene therapy of the present invention, the viral vector is a lentiviral vector.

Moreover, the expression vector for producing a cell for *ex vivo* gene therapy of the present invention includes a polynucleotide that encodes at least an amino acid sequence of a variant of human FVIII having the amino acid sequence at positions 20 to 2351 of the amino acid sequence of SEQ ID NO: 1 in which the B domain (the amino acid sequence at positions 760 to 1667) has been modified, thereby, the number of Asn residues subjected to N-linked glycosylation is four to six from the N-terminus and the function of the furin recognition site is lacking.

Moreover, the FVIII variant of the present invention is a variant of human FVIII having the amino acid sequence at positions 20 to 2351 of the amino acid sequence of SEQ ID NO: 1: and in the FVIII variant, the B domain (the amino acid sequence at positions 760 to 1667) has been modified, the number of Asn residues subjected to N-linked glycosylation is four to six from the N-terminus, the function of the furin recognition site is lacking and the Pro residue at position 758 has been substituted with another amino acid residue.

Moreover, in the FVIII variant of the present invention, the other amino acid residue is a polar uncharged amino acid residue selected from Thr residue, Ser residue, Asn residue and Gln residue.

Moreover, the polynucleotide of the present invention encodes at least an amino acid sequence of the FVIII variant of the present invention.

Moreover, the composition for transplantation of the present invention further contains a cell that produces human vWF and/or a fragment thereof *in vivo.*

Moreover, the cell of the present invention produces a fragment of human vWF *in vivo.*

Moreover, in the cell of the present invention, the fragment of human vWF is a fragment containing at least the D' domain and the D3 domain, which form a binding site for FVIII.

Moreover, in the cell of the present invention, the fragment of human vWF is a fragment which has the SP, D' and D3 domains, does not have any of the A2, A3, D4, B1, B2, B3, C1 and C2 domains, may have 50 amino acid residues maximum from the N-terminus of the A1 domain and may have but does not have to have the D1, D2 and CK domains of a domain structure SP-D1-D2-D'-D3-A1-A2-A3-D4-B1-B2-B3-C1-C2-CK which a precursor has.

Moreover, in the cell of the present invention, the fragment of human vWF is any of (a) to (c) below.
(a) a protein having the amino acid sequence of SEQ ID NO: 29
(b) a protein which has an amino acid sequence containing deletion, substitution, addition or insertion of one or a plurality of amino acid residues in the amino acid sequence of SEQ ID NO: 29 and which has an equivalent biological activity to that of the protein in (a)
(c) a protein which has an amino acid sequence having a sequence identity of at least 90% with the amino acid sequence of SEQ ID NO: 29 and which has an equivalent biological activity to that of the protein in (a)

Moreover, in the cell of the present invention, the fragment of human vWF is any of (a) to (c) below.
(a) a protein having the amino acid sequence of SEQ ID NO: 30
(b) a protein which has an amino acid sequence containing deletion, substitution, addition or insertion of one or a plurality of amino acid residues in the amino acid sequence of SEQ ID NO: 30 and which has an equivalent biological activity to that of the protein in (a)
(c) a protein which has an amino acid sequence having a sequence identity of at least 90% with the amino acid sequence of SEQ ID NO: 30 and which has an equivalent biological activity to that of the protein in (a)

Moreover, in the cell of the present invention, the fragment of human vWF is any of (a) to (c) below.
(a) a protein having the amino acid sequence of SEQ ID NO: 31
(b) a protein which has an amino acid sequence containing deletion, substitution, addition or insertion of one or a plurality of amino acid residues in the amino acid sequence of SEQ ID NO: 31 and which has an equivalent biological activity to that of the protein in (a)
(c) a protein which has an amino acid sequence having a sequence identity of at least 90% with the amino acid sequence of SEQ ID NO: 31 and which has an equivalent biological activity to that of the protein in (a)

Moreover, the drug of the present invention is for increasing the secretion amount of the FVIII variant produced by transplanting the cell for *ex vivo* gene therapy of the present invention into the body of a subject of the *ex vivo* gene therapy into blood and contains a cell that produces human vWF and/or a fragment thereof *in vivo* as an active ingredient.

Moreover, the composition for transplantation of the present invention contains a cell that produces human vWF and/or a fragment thereof *in vivo* and a pharmaceutically acceptable carrier.

Moreover, the set of compositions for transplantation of the present invention contains the composition for transplantation containing the cell for *ex vivo* gene therapy and a pharmaceutically acceptable carrier of the present invention and the composition for transplantation containing the cell that produces human vWF and/or a fragment thereof *in vivo* and a pharmaceutically acceptable carrier of the present invention.

### Effect of the Invention

According to the present invention, a novel cell for *ex vivo* gene therapy which produces a FVIII variant can be provided.

### Brief Description of the Drawings

[FIG. 1] A figure showing the differences in the amino acid sequence of the B domain and the sequences before and after the domain among the five types of FVIII variant (ΔB, N6, N6Δfurin, N5Δfurin and N5Δfurin (P739T)) in Experiment 1 of Experiment A in Examples.
[FIG. 2] The structure of the N5Δfurin (P739T)-producing lentiviral vector plasmid in the experiment.
[FIG. 3] A graph showing the average transgene copy numbers of the FVIII variant genes in the produced five types of FVIII variant gene-introduced preadipocytes in Experiment 2 of Experiment A in Examples.
[FIG. 4] A graph showing the FVIII antigen amounts in the culture solutions of the produced five types of FVIII variant gene-introduced preadipocytes in the experiment.
[FIG. 5] A graph showing the FVIII activities of the culture solutions of the produced five types of FVIII variant gene-introduced preadipocytes in the experiment.
[FIG. 6] A graph showing the specific activities (activity per antigen amount) of the FVIII variants secreted into the culture solutions from the produced five types of FVIII variant gene-introduced preadipocytes in the experiment.
[FIG. 7] A graph showing the FVIII antigen amounts in the bloods of the mice to which the produced five types of FVIII variant gene-introduced preadipocytes were transplanted subcutaneously into the backs in Experiment 4 of Experiment A in Examples.
[FIG. 8] A graph showing the average transgene copy numbers of the FVIII variant genes in the produced seven types of FVIII variant gene-introduced preadipocytes in Experiment 2 of Experiment B in Examples.
[FIG. 9] A graph showing the FVIII antigen amounts in the culture solutions of the produced seven types of FVIII variant gene-introduced preadipocytes in the experiment.
[FIG. 10] A graph showing the FVIII activities of the culture solutions of the produced seven types of FVIII variant gene-introduced preadipocytes in the experiment.
[FIG. 11] A graph showing the specific activities (activity per antigen amount) of the FVIII variants secreted into the culture solutions from the produced seven types of FVIII variant gene-introduced preadipocytes in the experiment.
[FIG. 12] The structure of the vWF3-producing lentiviral vector plasmid in Experiment 1 of Experiment C in Examples.
[FIG. 13] A graph showing the FVIII antigen amounts in the bloods of the mice to which the produced three types of human vWF fragment gene-introduced preadipocytes were transplanted as mixtures with the N5Δfurin (P739T) gene-introduced preadipocytes subcutaneously into the backs in Experiment 2 of Experiment C in Examples.
[FIG. 14] A graph showing the FVIII antigen amounts in the bloods of the mice to which the vWF3 gene-introduced preadipocytes were transplanted as mixtures with the N5Δfurin (P739T) gene-introduced preadipocytes subcutaneously into the backs using atelocollagen as a scaffold material in Experiment 3 of Experiment C in Examples.
[FIG. 15] A graph showing the FVIII antigen amounts in the bloods of the mice to which the vWF3 gene-introduced preadipocytes were transplanted as mixtures with the N5Δfurin (P739T) gene-introduced preadipocytes subcutaneously into the backs or inguinally in Experiment 4 of Experiment C in Examples.
[FIG. 16] A graph showing the FVIII antigen amounts in the bloods of the mice to which the vWF3 gene-introduced preadipocytes were transplanted as mixtures with the N5Δfurin (P739T) gene-introduced preadipocytes at three ratios of transplanted cell counts inguinally in Experiment 5 of Experiment C in Examples.

### Mode for Carrying Out the Invention

The cell for *ex vivo* gene therapy of the present invention produces a variant of human FVIII having the amino acid sequence at positions 20 to 2351 of the amino acid sequence of SEQ ID NO: 1 in which the B domain (the amino acid sequence at positions 760 to 1667) has been modified, thereby, the number of Asn residues subjected to N-linked glycosylation is four to six from the N-terminus and the function of the furin recognition site is lacking.

The FVIII variant in the present invention is a variant of human FVIII (a full-length form composed of 2332 amino acid residues) having the amino acid sequence at positions 20 to 2351 of the amino acid sequence of SEQ ID NO: 1 in which the B domain (the amino acid sequence at positions 760 to 1667) has been modified, thereby, which has the two structural features below.
(A) The number of Asn residues subjected to N-linked glycosylation is four to six from the N-terminus.
(B) The function of the furin recognition site is lacking.

FVIII variants having the structural feature (A) are known in the document of Hongzhi Z. Miao et al. (Bioengineering of coagulation factor VIII for improved secretion. Blood. 2004; 103: 3412-3419). The B domain of human FVIII has 19 Asn residues subjected to N-linked glycosylation, and the document reports that, when genes of FVIII variants obtained by modifying the B domain into amino acid sequences in such a manner that the number of Asn residues subjected to N-linked glycosylation increased one by one from the N-terminus of the B domain were introduced into COS-1 cells and the FVIII variants were produced *in vitro,* the secretion amount of the variant increased as the number of Asn residues increased, until the variant (N8) having an amino acid sequence having eight Asn residues (however, the secretion amount of the variant (N7) having an amino acid sequence having seven Asn residues is not described).

A FVIII variant having the structural feature (B) is known in the document of G. N. Nguyen et al. (Novel factor VIII variants with a modified furin cleavage site improve the efficacy of gene therapy for hemophilia A. Journal of Thrombosis and Haemostasis. 2017; 15: 110-121). The document reports that when the gene of the FVIII variant (human FVIII-BDDΔfurin), in which the furin recognition site composed of the four amino acid residues from the C-terminus of the B domain (Arg1645-His1646-Gln1647-Arg1648 of the full-length form) of the FVIII variant (human FVIII-BDD) maintaining only the three amino acid residues from the N-terminus of the B domain of human FVIII and the eleven amino acid residues from the C-terminus (both including no Asn residue subjected to N-linked glycosylation) was mutated to lack the function thereof and thus to prevent the cleavage between Arg1648 at the C-terminus of the B domain and Glu1649 at the N-terminus of the a3 linker region by furin, was introduced to a hemophilia A/CD4 knockout mouse using an adeno-associated viral vector to produce human FVIII-BDDΔfurin *in vivo,* the secretion amount was higher than the secretion amount of a case in which human FVIII-BDD was produced.

However, the document of Hongzhi Z. Miao *et al.* does not disclose that the FVIII variants having the structural feature (A) are used for *ex vivo* gene therapy, and the document of G. N. Nguyen et *al.* does not disclose that the FVIII variant having the structural feature (B) is used for *ex vivo* gene therapy.

Moreover, JP2019-527541A discloses a FVIII variant having both of the structural feature (A) and the structural feature (B) and mentions that a polynucleotide encoding the amino acid sequence thereof is used for gene therapy, but specific explanation or evidences are not provided.

In the present invention, that "the number of Asn residues subjected to N-linked glycosylation is four to six from the N-terminus" in the structural feature (A) corresponds to variant N4, variant N5 and variant N6 in the document of Hongzhi Z. Miao *et al.*

In the present invention, that "the function of the furin recognition site is lacking" in the structural feature (B) is not specifically restricted as long as it is an aspect which prevents the cleavage between Arg1648 at the C-terminus of the B domain and Glu1649 at the N-terminus of the a3 linker region by furin, and includes an aspect in which at least one of the amino acid residues constituting the furin recognition site composed of the four amino acid residues from the C-terminus of the B domain is deleted, an aspect in which at least one thereof is substituted with one, two or more other amino acid residues and the like.

A specific example of the FVIII variant having both of the structural feature (A) and the structural feature (B) in the present invention is a protein (abbreviation: N4Δfurin) having the amino acid sequence at positions 20 to 1607 of the amino acid sequence of SEQ ID NO: 2, in which the number of Asn residues subjected to N-linked glycosylation in the structural feature (A) is four from the N-terminus.

Another specific example thereof is a protein (abbreviation: N5Δfurin) having the amino acid sequence at positions 20 to 1652 of the amino acid sequence of SEQ ID NO: 3, in which the number of Asn residues subjected to N-linked glycosylation in the structural feature (A) is five from the N-terminus.

Another specific example thereof is a protein (abbreviation: N6Δfurin) having the amino acid sequence at positions 20 to 1670 of the amino acid sequence of SEQ ID NO: 4, in which the number of Asn residues subjected to N-linked glycosylation in the structural feature (A) is six from the N-terminus.

Moreover, in the FVIII variant in the present invention, the Pro residue at position 758 of the amino acid sequence of SEQ ID NO: 1 may have been substituted with another amino acid residue. The Pro residue at position 758 of the amino acid sequence of SEQ ID NO: 1 (Pro739 in the full-length form) is located in the thrombin recognition site, which is for the cleavage between Arg740 at the C-terminus of the a2 linker region and Ser741 at the N-terminus of the B domain by thrombin. By substituting the Pro residue, for example, with a polar uncharged amino acid residue such as Thr residue, Ser residue, Asn residue and Gln residue, the secretion amount into blood can be increased (the blood concentration can be increased), or the specific activity (activity per antigen amount) can be increased.

A specific example of the FVIII variant which has both of the structural feature (A) and the structural feature (B) and in which the Pro residue at position 758 of the amino acid sequence of SEQ ID NO: 1 has been substituted with Thr residue is a protein (abbreviation: N5Δfurin (P739T)) having the amino acid sequence at positions 20 to 1652 of the amino acid sequence of SEQ ID NO: 5, in which the number of Asn residues subjected to N-linked glycosylation in the structural feature (A) is five from the N-terminus. A specific example of the FVIII variant which has both of the structural feature (A) and the structural feature (B) and in which the Pro residue at position 758 of the amino acid sequence of SEQ ID NO: 1 has been substituted with Ser residue is a protein (abbreviation: N5Δfurin (P739S)) having the amino acid sequence at positions 20 to 1652 of the amino acid sequence of SEQ ID NO: 14, in which the number of Asn residues subjected to N-linked glycosylation in the structural feature (A) is five from the N-terminus. A specific example of the FVIII variant which has both of the structural feature (A) and the structural feature (B) and in which the Pro residue at position 758 of the amino acid sequence of SEQ ID NO: 1 has been substituted with Asn residue is a protein (abbreviation: N5Δfurin (P739N)) having the amino acid sequence at positions 20 to 1652 of the amino acid sequence of SEQ ID NO: 15, in which the number of Asn residues subjected to N-linked glycosylation in the structural feature (A) is five from the N-terminus. A specific example of the FVIII variant which has both of the structural feature (A) and the structural feature (B) and in which the Pro residue at position 758 of the amino acid sequence of SEQ ID NO: 1 has been substituted with Gln residue is a protein (abbreviation: N5Δfurin (P739Q)) having the amino acid sequence at positions 20 to 1652 of the amino acid sequence of SEQ ID NO: 16, in which the number of Asn residues subjected to N-linked glycosylation in the structural feature (A) is five from the N-terminus.

The FVIII variants in the present invention include proteins which have deletion, substitution, addition or insertion of one or a plurality of amino acid residues in the respective amino acid sequences of the amino acid sequence at positions 20 to 1607 of the amino acid sequence of SEQ ID NO: 2, the amino acid sequence at positions 20 to 1652 of the amino acid sequence of SEQ ID NO: 3, the amino acid sequence at positions 20 to 1670 of the amino acid sequence of SEQ ID NO: 4, the amino acid sequence at positions 20 to 1652 of the amino acid sequence of SEQ ID NO: 5, the amino acid sequence at positions 20 to 1652 of the amino acid sequence of SEQ ID NO: 14, the amino acid sequence at positions 20 to 1652 of the amino acid sequence of SEQ ID NO: 15 and the amino acid sequence at positions 20 to 1652 of the amino acid sequence of SEQ ID NO: 16 and which have equivalent biological activities to those of the proteins having the respective amino acid sequences of the amino acid sequence at positions 20 to 1607 of the amino acid sequence of SEQ ID NO: 2, the amino acid sequence at positions 20 to 1652 of the amino acid sequence of SEQ ID NO: 3, the amino acid sequence at positions 20 to 1670 of the amino acid sequence of SEQ ID NO: 4, the amino acid sequence at positions 20 to 1652 of the amino acid sequence of SEQ ID NO: 5, the amino acid sequence at positions 20 to 1652 of the amino acid sequence of SEQ ID NO: 14, the amino acid sequence at positions 20 to 1652 of the amino acid sequence of SEQ ID NO: 15 and the amino acid sequence at positions 20 to 1652 of the amino acid sequence of SEQ ID NO: 16. Moreover, the FVIII variants include proteins which have amino acid sequences having a sequence identity of at least 90% with the respective amino acid sequences of the amino acid sequence at positions 20 to 1607 of the amino acid sequence of SEQ ID NO: 2, the amino acid sequence at positions 20 to 1652 of the amino acid sequence of SEQ ID NO: 3, the amino acid sequence at positions 20 to 1670 of the amino acid sequence of SEQ ID NO: 4, the amino acid sequence at positions 20 to 1652 of the amino acid sequence of SEQ ID NO: 5, the amino acid sequence at positions 20 to 1652 of the amino acid sequence of SEQ ID NO: 14, the amino acid sequence at positions 20 to 1652 of the amino acid sequence of SEQ ID NO: 15 and the amino acid sequence at positions 20 to 1652 of the amino acid sequence of SEQ ID NO: 16 and which have equivalent biological activities to those of the proteins having the respective amino acid sequences of the amino acid sequence at positions 20 to 1607 of the amino acid sequence of SEQ ID NO: 2, the amino acid sequence at positions 20 to 1652 of the amino acid sequence of SEQ ID NO: 3, the amino acid sequence at positions 20 to 1670 of the amino acid sequence of SEQ ID NO: 4, the amino acid sequence at positions 20 to 1652 of the amino acid sequence of SEQ ID NO: 5, the amino acid sequence at positions 20 to 1652 of the amino acid sequence of SEQ ID NO: 14, the amino acid sequence at positions 20 to 1652 of the amino acid sequence of SEQ ID NO: 15 and the amino acid sequence at positions 20 to 1652 of the amino acid sequence of SEQ ID NO: 16. Here, the "plurality" is an integer of 50 or less, preferably an integer of 30 or less, more preferably an integer of 20 or less, further preferably an integer of 10 or less, for example two to nine, two to seven, two to five or two or three. The sequence identity with the respective amino acid sequences of the amino acid sequence at positions 20 to 1607 of the amino acid sequence of SEQ ID NO: 2, the amino acid sequence at positions 20 to 1652 of the amino acid sequence of SEQ ID NO: 3, the amino acid sequence at positions 20 to 1670 of the amino acid sequence of SEQ ID NO: 4, the amino acid sequence at positions 20 to 1652 of the amino acid sequence of SEQ ID NO: 5, the amino acid sequence at positions 20 to 1652 of the amino acid sequence of SEQ ID NO: 14, the amino acid sequence at positions 20 to 1652 of the amino acid sequence of SEQ ID NO: 15 and the amino acid sequence at positions 20 to 1652 of the amino acid sequence of SEQ ID NO: 16 is preferably at least 91%, more preferably at least 92%, more preferably at least 93%, more preferably at least 94%, more preferably at least 95%, more preferably at least 96%, more preferably at least 97%, more preferably at least 98%, further preferably at least 99%. Here, the % of the identity refers to a value calculated using software for computing an identity between a plurality (two) of amino acid sequences (for example, FASTA, DANASYS, BLAST and the like) with default settings. The "equivalent biological activity" means that, for example, a biological activity such as expression of the function as a blood coagulation factor that FVIII has, activation thereof and inactivation thereof is equivalent.

The cells used for producing the cell for *ex vivo* gene therapy of the present invention are not specifically restricted as long as the cells can produce the FVIII variant in the present invention in the body (expressed as a protein bonded to the signal peptide having the amino acid sequence at positions 1 to 19 of the amino acid sequence of SEQ ID NO: 1) when the cells are transplanted into the body of a subject of the *ex vivo* gene therapy, and a specific example thereof is adipocytes. Adipocytes include mature adipocytes, which are spherical cells storing fat and contain lipid droplets, and also preadipocytes, which have the capability of differentiating into mature adipocytes. The adipocytes may be autologous cells or allogeneic cells. The cells for *ex vivo* gene therapy using adipocytes are described in detail in JP4879867B, and thus, the subject matters described in JP4879867B can be referred to appropriately in the present invention.

The cell for *ex vivo* gene therapy of the present invention can be produced by introducing an expression vector having a polynucleotide that encodes at least the amino acid sequence of a variant of human FVIII having the amino acid sequence at positions 20 to 2351 of the amino acid sequence of SEQ ID NO: 1 in which the B domain (the amino acid sequence at positions 760 to 1667) has been modified, thereby, the number of Asn residues subjected to N-linked glycosylation is four to six from the N-terminus and the function of the furin recognition site is lacking into, for example, a cell which has been removed from a subject of the *ex vivo* gene therapy or a healthy individual, for example, into an adipocyte collected from adipose tissue by a known method.

As the method for introducing an expression vector having a polynucleotide that encodes at least the amino acid sequence of a variant of human FVIII having the amino acid sequence at positions 20 to 2351 of the amino acid sequence of SEQ ID NO: 1 in which the B domain (the amino acid sequence at positions 760 to 1667) has been modified, thereby, the number of Asn residues subjected to N-linked glycosylation is four to six from the N-terminus and the function of the furin recognition site is lacking into an adipocyte, any known method may be employed, but the expression vector is preferably a viral vector which is excellent in transgenesis efficiency into cells, maintenance of replication in the cells, stability, expression efficiency and the like. As the viral vector, a known vector such as a retroviral vector, a lentiviral vector, an adenoviral vector and an adeno-associated viral vector can be used, but of these, a lentiviral vector, which is highly safe and can express a gene stably for a long time because the target gene is efficiently inserted into the host cell genome, is preferably used. A gene can be introduced into target cells using a lentiviral vector by a known method, for example, by transfecting packaging cells such as 293T cells and COS cells with a human immunodeficiency virus type-1 (HIV-1) -derived lentiviral vector plasmid having an inserted target gene together with a packaging plasmid and then infecting the target cells with the lentiviral vector having the target gene obtained as a cell culture supernatant (protamine sulfate, polybrene or the like may be added to increase the infection efficiency) . Tools for producing a lentiviral vector having a target gene have also been commercialized (for example, those of Takara Bio Inc., Funakoshi Co., Ltd., Cosmo Bio Co., Ltd. and the like).

The lentiviral vector having a polynucleotide that encodes at least the amino acid sequence of a variant of human FVIII having the amino acid sequence at positions 20 to 2351 of the amino acid sequence of SEQ ID NO: 1 in which the B domain (the amino acid sequence at positions 760 to 1667) has been modified, thereby, the number of Asn residues subjected to N-linked glycosylation is four to six from the N-terminus and the function of the furin recognition site is lacking can be obtained by inserting a polynucleotide that encodes at least the amino acid sequence of a variant of human FVIII having the amino acid sequence at positions 20 to 2351 of the amino acid sequence of SEQ ID NO: 1 in which the B domain (the amino acid sequence at positions 760 to 1667) has been modified, thereby, the number of Asn residues subjected to N-linked glycosylation is four to six from the N-terminus and the function of the furin recognition site is lacking into a lentiviral vector plasmid as a target gene, transfecting packaging cells such as 293T cells and COS cells with the lentiviral vector plasmid having the target gene together with a packaging plasmid and then obtaining the lentiviral vector as a cell culture supernatant.

The polynucleotide that encodes at least the amino acid sequence of a variant of human FVIII having the amino acid sequence at positions 20 to 2351 of the amino acid sequence of SEQ ID NO: 1 in which the B domain (the amino acid sequence at positions 760 to 1667) has been modified, thereby, the number of Asn residues subjected to N-linked glycosylation is four to six from the N-terminus and the function of the furin recognition site is lacking is, for example, a polynucleotide that encodes the amino acid sequence of a protein in that a variant of human FVIII having the amino acid sequence at positions 20 to 2351 of the amino acid sequence of SEQ ID NO: 1 in which the B domain (the amino acid sequence at positions 760 to 1667) has been modified, thereby, the number of Asn residues subjected to N-linked glycosylation is four to six from the N-terminus and the function of the furin recognition site is lacking is bonded to the signal peptide having the amino acid sequence at positions 1 to 19 of the amino acid sequence of SEQ ID NO: 1. The polynucleotide that encodes the amino acid sequence of a protein in that a variant of human FVIII having the amino acid sequence at positions 20 to 2351 of the amino acid sequence of SEQ ID NO: 1 in which the B domain (the amino acid sequence at positions 760 to 1667) has been modified, thereby, the number of Asn residues subjected to N-linked glycosylation is four to six from the N-terminus and the function of the furin recognition site is lacking is bonded to the signal peptide having the amino acid sequence at positions 1 to 19 of the amino acid sequence of SEQ ID NO: 1 can be prepared, for example, by chemical synthesis based on the amino acid sequence of a protein in that a variant of human FVIII having the amino acid sequence at positions 20 to 2351 of the amino acid sequence of SEQ ID NO: 1 in which the B domain (the amino acid sequence at positions 760 to 1667) has been modified, thereby, the number of Asn residues subjected to N-linked glycosylation is four to six from the N-terminus and the function of the furin recognition site is lacking is bonded to the signal peptide having the amino acid sequence at positions 1 to 19 of the amino acid sequence of SEQ ID NO: 1 (the codons of the base sequence may be optimized according to the need).

Specific examples of the polynucleotide that encodes the amino acid sequence of a protein in that a variant of human FVIII having the amino acid sequence at positions 20 to 2351 of the amino acid sequence of SEQ ID NO: 1 in which the B domain (the amino acid sequence at positions 760 to 1667) has been modified, thereby, the number of Asn residues subjected to N-linked glycosylation is four to six from the N-terminus and the function of the furin recognition site is lacking is bonded to the signal peptide having the amino acid sequence at positions 1 to 19 of the amino acid sequence of SEQ ID NO: 1 include a polynucleotide having the base sequence of SEQ ID NO: 6 (a polynucleotide encoding the amino acid sequence of a protein in which N4Δfurin having the amino acid sequence at positions 1 to 1607 of the amino acid sequence of SEQ ID NO: 2 is bonded to the signal peptide), a polynucleotide having the base sequence of SEQ ID NO: 7 (a polynucleotide encoding the amino acid sequence of a protein in which N5Δfurin having the amino acid sequence at positions 1 to 1652 of the amino acid sequence of SEQ ID NO: 3 is bonded to the signal peptide), a polynucleotide having the base sequence of SEQ ID NO: 8 (a polynucleotide encoding the amino acid sequence of a protein in which N6Δfurin having the amino acid sequence at positions 1 to 1670 of the amino acid sequence of SEQ ID NO: 4 is bonded to the signal peptide), a polynucleotide having the base sequence of SEQ ID NO: 9 (a polynucleotide encoding the amino acid sequence of a protein in which N5Δfurin (P739T) having the amino acid sequence at positions 1 to 1652 of the amino acid sequence of SEQ ID NO: 5 is bonded to the signal peptide), a polynucleotide having the base sequence of SEQ ID NO: 17 (a polynucleotide encoding the amino acid sequence of a protein in which N5Δfurin (P739S) having the amino acid sequence at positions 1 to 1652 of the amino acid sequence of SEQ ID NO: 14 is bonded to the signal peptide), a polynucleotide having the base sequence of SEQ ID NO: 18 (a polynucleotide encoding the amino acid sequence of a protein in which N5Δfurin (P739N) having the amino acid sequence at positions 1 to 1652 of the amino acid sequence of SEQ ID NO: 15 is bonded to the signal peptide) and a polynucleotide having the base sequence of SEQ ID NO: 19 (a polynucleotide encoding the amino acid sequence of a protein in which N5Δfurin (P739Q) having the amino acid sequence at positions 1 to 1652 of the amino acid sequence of SEQ ID NO: 16 is bonded to the signal peptide). However, the polynucleotide that encodes the amino acid sequence of a protein in that a variant of human FVIII having the amino acid sequence at positions 20 to 2351 of the amino acid sequence of SEQ ID NO: 1 in which the B domain (the amino acid sequence at positions 760 to 1667) has been modified, thereby, the number of Asn residues subjected to N-linked glycosylation is four to six from the N-terminus and the function of the furin recognition site is lacking is bonded to the signal peptide having the amino acid sequence at positions 1 to 19 of the amino acid sequence of SEQ ID NO: 1 is not limited to these polynucleotides and includes polynucleotides which hybridize with the complementary strands of these polynucleotides under stringent conditions and which encode at least proteins having equivalent biological activities to those of the proteins having the respective amino acid sequences of the amino acid sequence at positions 20 to 1607 of the amino acid sequence of SEQ ID NO: 2, the amino acid sequence at positions 20 to 1652 of the amino acid sequence of SEQ ID NO: 3, the amino acid sequence at positions 20 to 1670 of the amino acid sequence of SEQ ID NO: 4, the amino acid sequence at positions 20 to 1652 of the amino acid sequence of SEQ ID NO: 5, the amino acid sequence at positions 20 to 1652 of the amino acid sequence of SEQ ID NO: 14, the amino acid sequence at positions 20 to 1652 of the amino acid sequence of SEQ ID NO: 15 and the amino acid sequence at positions 20 to 1652 of the amino acid sequence of SEQ ID NO: 16. Moreover, polynucleotides which have a sequence identity of at least 90%, preferably at least 91%, more preferably at least 92%, more preferably at least 93%, more preferably at least 94%, more preferably at least 95%, more preferably at least 96%, more preferably at least 97%, more preferably at least 98%, further preferably at least 99%, with the respective base sequences of SEQ ID NOs: 6 to 9 and 17 to 19 and which encode at least proteins having equivalent biological activities to those of the proteins having the respective amino acid sequences of the amino acid sequence at positions 20 to 1607 of the amino acid sequence of SEQ ID NO: 2, the amino acid sequence at positions 20 to 1652 of the amino acid sequence of SEQ ID NO: 3, the amino acid sequence at positions 20 to 1670 of the amino acid sequence of SEQ ID NO: 4, the amino acid sequence at positions 20 to 1652 of the amino acid sequence of SEQ ID NO: 5, the amino acid sequence at positions 20 to 1652 of the amino acid sequence of SEQ ID NO: 14, the amino acid sequence at positions 20 to 1652 of the amino acid sequence of SEQ ID NO: 15 and the amino acid sequence at positions 20 to 1652 of the amino acid sequence of SEQ ID NO: 16 are included. Here, the "hybridization under stringent conditions" includes, for example, hybridization at 30 to 50°C in 3 to 4 × SSC (150 mM sodium chloride and 15 mM sodium citrate, pH 7.2) and 0.1 to 0.5% SDS for one to 24 hours, preferably hybridization at 40 to 45°C in 3.4 × SSC and 0.3% SDS for one to 24 hours, and subsequent washing. As washing conditions, for example, conditions such as continuous washing with a solution containing 2 × SSC and 0.1% SDS, a 1 × SSC solution and a 0.2 × SSC solution at room temperature are exemplified. The combination of the above conditions, however, is an example, and one skilled in the art can achieve the same stringency as described above by appropriately combining the above elements and other elements which determine the stringency of hybridization (for example, the concentration, the length and the GC amount of the hybridization probe, the reaction time of hybridization and the like).

The cell for *ex vivo* gene therapy of the present invention produces a variant of human FVIII having the amino acid sequence at positions 20 to 2351 of the amino acid sequence of SEQ ID NO: 1 in which the B domain (the amino acid sequence at positions 760 to 1667) has been modified, thereby, the number of Asn residues subjected to N-linked glycosylation is four to six from the N-terminus and the function of the furin recognition site is lacking *in vivo* when the cell is transplanted into the body of a subject of the *ex vivo* gene therapy for hemophilia A, and thus the cell is useful for *ex vivo* gene therapy for hemophilia A. The transplanted cell count of the cells for *ex vivo* gene therapy of the present invention is determined considering the age, the weight, the gender, the disease state, the seriousness and the like of a subject of the *ex vivo* gene therapy.

When the cell for *ex vivo* gene therapy of the present invention is produced using an adipocyte, the cell can be transplanted by injecting into subcutaneous tissue or adipose tissue by a known method. At this point, the cell for *ex vivo* gene therapy of the present invention is preferably transplanted as a composition for transplantation together with a pharmaceutically acceptable carrier, such as physiological saline, Ringer's solution, phosphate buffer, a culture solution, serum and body fluid. The composition for transplantation may further contain various extracellular matrix components (for example, collagen, laminin, heparan sulfate and gelatin) or the like as a scaffold material. Moreover, in order that blood vessels are formed in the surrounding area after the transplantation and that the produced FVIII variant is secreted into blood with a high efficiency, the composition for transplantation can also contain an angiogenic factor (which may be naturally derived or produced by a genetic engineering method) such as vascular endothelial growth factor (VEGF), basic fibroblast growth factor (bFGF), acidic fibroblast growth factor (aFGF), platelet-derived growth factor (PDGF) and hepatocyte growth factor (HGF).

Moreover, the cell for *ex vivo* gene therapy of the present invention may be transplanted into the body of a subject of the *ex vivo* gene therapy together with a cell that produces human von Willebrand factor ("vWF" below), which contributes to stabilization and transport of FVIII in the circulating blood, or a fragment thereof (for example, a fragment containing at least the D' domain and the D3 domain, which form a binding site for FVIII) *in vivo.* Interestingly, when the cell for *ex vivo* gene therapy of the present invention is transplanted into the body of a subject of the *ex vivo* gene therapy together with a cell that produces human vWF or a fragment thereof *in vivo,* the cell that produces human vWF or a fragment thereof *in vivo* exhibits the effect of increasing the secretion amount of the FVIII variant produced by the cell for *ex vivo* gene therapy of the present invention into blood (increasing the blood concentration).

Human vWF is a glycoprotein which is expressed in vascular endothelial cells and bone marrow megakaryocytes as a precursor (the amino acid sequence is shown in SEQ ID NO: 28) composed of 2813 amino acid residues including a signal peptide composed of 22 amino acid residues. The precursor has a domain structure SP-D1-D2-D'-D3-A1-A2-A3-D4-B1-B2-B3-C1-C2-CK and turns into pro-vWF composed of a propeptide composed of 741 amino acid residues (D1-D2) and a mature subunit composed of 2050 amino acid residues (D'-D3-A1-A2-A3-D4-B1-B2-B3-C1-C2-CK) when the signal peptide (SP) is cleaved. Pro-vWF exhibits certain functions after undergoing dimer formation through S-S binding at the C-terminus, subsequent multimer formation through S-S binding of dimers at the N-termini, glycosylation, cleavage of the propeptide and the like.

The cell that produces a fragment of human vWF containing at least the D' domain and the D3 domain, which form a binding site for FVIII, *in vivo* and that may be transplanted into the body of a subject of the *ex vivo* gene therapy together with the cell for *ex vivo* gene therapy of the present invention is, for example, a cell that produces a fragment which has the SP, D' and D3 domains, does not have any of the A2, A3, D4, B1, B2, B3, C1 and C2 domains, may have 50 amino acid residues maximum from the N-terminus of the A1 domain and may have but does not have to have the D1, D2 and CK domains of the domain structure SP-D1-D2-D'-D3-A1-A2-A3-D4-B1-B2-B3-C1-C2-CK which the precursor has *in vivo.* Here, the amino acid sequences of the SP, D1, D2, D', D3, A1 and CK domains are the amino acid sequence at positions 1 to 22, the amino acid sequence at positions 23 to 386, the amino acid sequence at positions 387 to 763, the amino acid sequence at positions 764 to 864, the amino acid sequence at positions 865 to 1242, the amino acid sequence at positions 1243 to 1479 and the amino acid sequence at positions 2664 to 2813, respectively, in the amino acid sequence of SEQ ID NO: 28 which the precursor has.

A specific example of the fragment which has the SP, D' and D3 domains, does not have any of the A2, A3, D4, B1, B2, B3, C1 and C2 domains, may have 50 amino acid residues maximum from the N-terminus of the A1 domain and may have but does not have to have the D1, D2 and CK domains of the domain structure SP-D1-D2-D'-D3-A1-A2-A3-D4-B1-B2-B3-C1-C2-CK, which the precursor has, is a fragment composed of the SP, D1, D2, D' and D3 domains, 26 amino acid residues from the N-terminus of the A1 domain, additional three amino acid residues and the CK domain (a protein having the amino acid sequence of SEQ ID NO: 29, abbreviation: vWF1). The "26 amino acid residues from the N-terminus of the A1 domain" correspond to the amino acid residues constituting the amino acid sequence at positions 1243 to 1268 of the amino acid sequence of SEQ ID NO: 28, which the precursor has, and include a plurality of amino acid residues (serine and threonine) to which an O-linked oligosaccharide binds. The "additional three amino acid residues" correspond to the amino acid residues at positions 1269, 1270 and 2663 of the amino acid sequence of SEQ ID NO: 28 which the precursor has. That is, the amino acid sequence of vWF1 is the amino acid sequence in which the amino acid sequence at positions 1 to 1270 and the amino acid sequence at positions 2663 to 2813 of the amino acid sequence of SEQ ID NO: 28 which the precursor has are linked. Moreover, the fragment which has the SP, D' and D3 domains, does not have any of the A2, A3, D4, B1, B2, B3, C1 and C2 domains, may have 50 amino acid residues maximum from the N-terminus of the A1 domain and may have but does not have to have the D1, D2 and CK domains of the domain structure SP-D1-D2-D'-D3-A1-A2-A3-D4-B1-B2-B3-C1-C2-CK which the precursor has may be a fragment composed of the SP, D1, D2, D' and D3 domains and 26 amino acid residues from the N-terminus of the A1 domain (a protein having the amino acid sequence of SEQ ID NO: 30, abbreviation: vWF2), a fragment composed of the SP, D' and D3 domains and 26 amino acid residues from the N-terminus of the A1 domain (a protein having the amino acid sequence of SEQ ID NO: 31, abbreviation vWF3) or the like.

The fragment of human vWF includes, for example, proteins which have deletion, substitution, addition or insertion of one or a plurality of amino acid residues in the amino acid sequence of SEQ ID NO: 29, the amino acid sequence of SEQ ID NO: 30 and the amino acid sequence of SEQ ID NO: 31 and which have equivalent biological activities to those of the proteins having the respective amino acid sequences of the amino acid sequence of SEQ ID NO: 29, the amino acid sequence of SEQ ID NO: 30 and the amino acid sequence of SEQ ID NO: 31. Moreover, the fragment of human vWF includes proteins which have amino acid sequences having a sequence identity of at least 90% with the respective amino acid sequences of the amino acid sequence of SEQ ID NO: 29, the amino acid sequence of SEQ ID NO: 30 and the amino acid sequence of SEQ ID NO: 31 and which have equivalent biological activities to those of the proteins having the respective amino acid sequences of the amino acid sequence of SEQ ID NO: 29, the amino acid sequence of SEQ ID NO: 30 and the amino acid sequence of SEQ ID NO: 31. Here, the "plurality" is an integer of 50 or less, preferably an integer of 30 or less, more preferably an integer of 20 or less, further preferably an integer of 10 or less, for example two to nine, two to seven, two to five or two or three. The sequence identity with the respective amino acid sequences of the amino acid sequence of SEQ ID NO: 29, the amino acid sequence of SEQ ID NO: 30 and the amino acid sequence of SEQ ID NO: 31 is preferably at least 91%, more preferably at least 92%, more preferably at least 93%, more preferably at least 94%, more preferably at least 95%, more preferably at least 96%, more preferably at least 97%, more preferably at least 98%, further preferably at least 99%. Here, the % of the identity refers to a value calculated using software for computing an identity between a plurality (two) of amino acid sequences (for example, FASTA, DANASYS, BLAST and the like) with default settings. The "equivalent biological activity" means that, for example, a biological activity such as the function as vWF (for example, stabilization and transport of FVIII in the circulating blood) is equivalent.

The cell that produces human vWF or a fragment thereof *in vivo* and that may be transplanted into the body of a subject of the *ex vivo* gene therapy together with the cell for *ex vivo* gene therapy of the present invention can be produced by introducing an expression vector having a polynucleotide that encodes at least the amino acid sequence of human vWF or a fragment thereof into, for example, a cell which has been removed from a subject of the *ex vivo* gene therapy or a healthy individual, for example, into an adipocyte collected from adipose tissue by a known method, according to the method for producing the cell for *ex vivo* gene therapy of the present invention described above.

Specific examples of the polynucleotide that encodes the amino acid sequence of a fragment of human vWF include a polynucleotide having the base sequence of SEQ ID NO: 32 (a polynucleotide encoding the amino acid sequence of SEQ ID NO: 29), a polynucleotide having the base sequence of SEQ ID NO: 33 (a polynucleotide encoding the amino acid sequence of SEQ ID NO: 30) and a polynucleotide having the base sequence of SEQ ID NO: 34 (a polynucleotide encoding the amino acid sequence of SEQ ID NO: 31). However, the polynucleotide that encodes the amino acid sequence of a fragment of human vWF is not limited to these polynucleotides and includes polynucleotides which hybridize with the complementary strands of these polynucleotides under stringent conditions and which encode at least proteins having equivalent biological activities to those of the proteins having the respective amino acid sequences of the amino acid sequence of SEQ ID NO: 29, the amino acid sequence of SEQ ID NO: 30 and the amino acid sequence of SEQ ID NO: 31. Moreover, polynucleotides which have a sequence identity of at least 90%, preferably at least 91%, more preferably at least 92%, more preferably at least 93%, more preferably at least 94%, more preferably at least 95%, more preferably at least 96%, more preferably at least 97%, more preferably at least 98%, further preferably at least 99%, with the respective base sequences of SEQ ID NOs: 32 to 34 and which encode at least proteins having equivalent biological activities to those of the proteins having the respective amino acid sequences of the amino acid sequence of SEQ ID NO: 29, the amino acid sequence of SEQ ID NO: 30 and the amino acid sequence of SEQ ID NO: 31 are included. Here, the "hybridization under stringent conditions" includes, for example, hybridization at 30 to 50°C in 3 to 4 × SSC (150 mM sodium chloride and 15 mM sodium citrate, pH 7.2) and 0.1 to 0.5% SDS for one to 24 hours, preferably hybridization at 40 to 45°C in 3.4 × SSC and 0.3% SDS for one to 24 hours, and subsequent washing. As washing conditions, for example, conditions such as continuous washing with a solution containing 2 × SSC and 0.1% SDS, a 1 × SSC solution and a 0.2 × SSC solution at room temperature are exemplified. The combination of the above conditions, however, is an example, and one skilled in the art can achieve the same stringency as described above by appropriately combining the above elements and other elements which determine the stringency of hybridization (for example, the concentration, the length and the GC amount of the hybridization probe, the reaction time of hybridization and the like).

As the method for transplanting the cell that produces human vWF or a fragment thereof *in vivo* into the body of a subject of the *ex vivo* gene therapy together with the cell for *ex vivo* gene therapy of the present invention, for example, a method of transplanting the composition for transplantation containing the cell for *ex vivo* gene therapy of the present invention and a pharmaceutically acceptable carrier described above to which the cell that produces human vWF or a fragment thereof *in vivo* is further added is exemplified. However, a composition for transplantation containing the cell that produces human vWF or a fragment thereof *in vivo* and a pharmaceutically acceptable carrier may be prepared separately from the composition for transplantation containing the cell for *ex vivo* gene therapy of the present invention and a pharmaceutically acceptable carrier, and the two kinds of composition for transplantation may be transplanted as a set simultaneously or with an interval. In this case, the pharmaceutically acceptable carrier in the composition for transplantation containing the cell that produces human vWF or a fragment thereof *in vivo* and a pharmaceutically acceptable carrier may be the same as the pharmaceutically acceptable carrier in the composition for transplantation containing the cell for *ex vivo* gene therapy of the present invention and a pharmaceutically acceptable carrier. Moreover, the composition for transplantation containing the cell that produces human vWF or a fragment thereof *in vivo* and a pharmaceutically acceptable carrier can contain various extracellular matrix components, angiogenic factors or the like, as the composition for transplantation containing the cell for *ex vivo* gene therapy of the present invention and a pharmaceutically acceptable carrier.

When the cell that produces human vWF or a fragment thereof *in vivo* is transplanted into the body of a subject of the *ex vivo* gene therapy together with the cell for ex *vivo* gene therapy of the present invention, the transplanted cell count ratio of the cells for *ex vivo* gene therapy of the present invention and the cells that produce human vWF or a fragment thereof *in vivo* is determined, for example, from the range of 1:100 to 100:1, considering the transgene copy numbers and the like in the both.

### Examples

Hereinafter, the present invention is explained in detail with Examples, but the present invention should not be construed as being limited to the descriptions below.

### Experiment A: Examination of Various FVIII Variant Gene-Introduced Preadipocytes (1)

### Experiment 1: Production of FVIII Variant Gene-Introduced Preadipocytes

### (1) Production of FVIII Variant-Producing Lentiviral Vector Plasmids

Polynucleotides in which the restriction enzyme sites for SbfI and MluI were added to the 5' terminus and the 3' terminus, respectively, of base sequences corresponding to the amino acid sequences of the proteins composed of the respective five types of FVIII variant below and a signal peptide bonded thereto were synthesized and inserted into the SbfI/MluI site of pLVSIN-EF1α Neo Vector (Takara Bio Inc., the same applies below), which is a SIN-type lentiviral vector, and thus FVIII variant-producing lentiviral vector plasmids were produced. In this regard, the codons of the respective base sequences were optimized using GeneArt GeneOptimizer software (Thermo Fisher Scientific, Inc.) based on the respective amino acid sequences.
(a) a base sequence (SEQ ID NO: 11) corresponding to the amino acid sequence (SEQ ID NO: 10, which maintains the function of the furin recognition site but lacks almost the entire B domain) of a protein in which the signal peptide is bonded to a gene recombinant B domain-deleted human FVIII preparation, NovoEight (Novo Nordisk Pharma Ltd., Eur J Haematol. 2014 Nov; 93(5): 369-376) (ΔB)
(b) a base sequence (SEQ ID NO: 13) corresponding to the amino acid sequence (SEQ ID NO: 12) of a protein in that the signal peptide is bonded to a FVIII variant (N6) in which the number of Asn residues subjected to N-linked glycosylation in the B domain of human FVIII is six from the N-terminus and which maintains the function of the furin recognition site
(c) a base sequence (SEQ ID NO: 8) corresponding to the amino acid sequence (SEQ ID NO: 4) of a protein in that the signal peptide is bonded to a FVIII variant (N6Δfurin) in which the number of Asn residues subjected to N-linked glycosylation in the B domain of human FVIII is six from the N-terminus and which lacks the function of the furin recognition site
(d) a base sequence (SEQ ID NO: 7) corresponding to the amino acid sequence (SEQ ID NO: 3) of a protein in that the signal peptide is bonded to a FVIII variant (N5Δfurin) in which the number of Asn residues subjected to N-linked glycosylation in the B domain of human FVIII is five from the N-terminus and which lacks the function of the furin recognition site
(e) a base sequence (SEQ ID NO: 9) corresponding to the amino acid sequence (SEQ ID NO: 5) of a protein in that the signal peptide is bonded to a FVIII variant (N5Δfurin (P739T)) in which the number of Asn residues subjected to N-linked glycosylation in the B domain of human FVIII is five from the N-terminus and which lacks the function of the furin recognition site and has substitution of the Pro residue at position 758 with a Thr residue

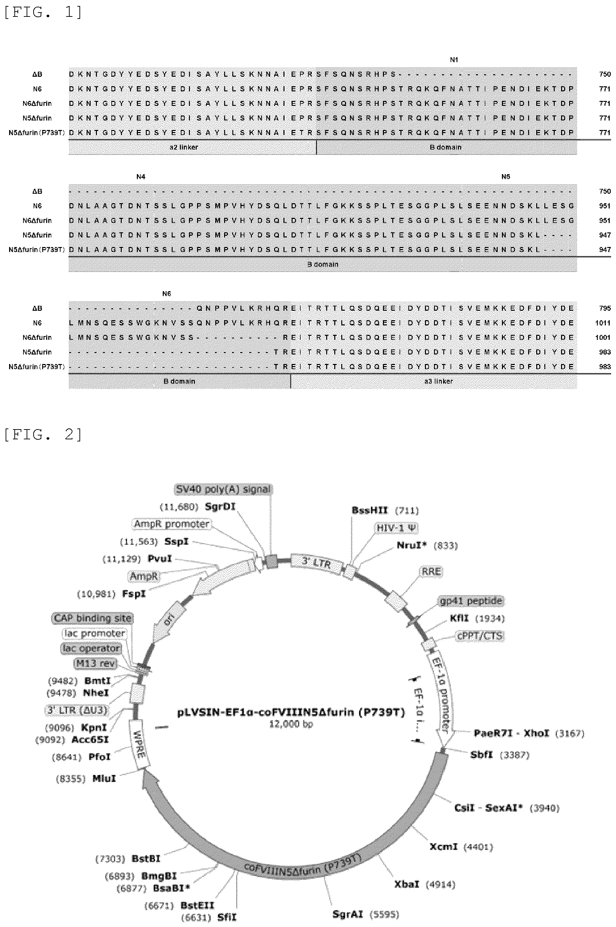

The differences (the positions in the full-length form) in the amino acid sequence of the B domain and the sequences before and after the domain among the five types of FVIII variant above are shown in FIG. 1. Moreover, the structure of the N5Δfurin (P739T)-producing lentiviral vector plasmid is shown in FIG. 2.

### (2) Production of FVIII Variant-Producing Lentiviral Vectors

The FVIII variant-producing lentiviral vector plasmids produced in (1) (5.5 µg), serum-free DMEM (1.5 mL), Lentiviral High Titer Packaging Mix (Takara Bio Inc., 7 µL) and PEIpro transfection reagent (Avantor, Inc., 45 µL) were gently mixed by pipetting and left still for 15 minutes at room temperature, and thus transfection solutions were prepared. The transfection solutions were added dropwise to Lenti-X293T cells (Takara Bio Inc.) which were seeded and cultured at 1×10⁷ cells/10 mL in 10% FBS-containing DMEM/10 cm dishes from about 24 hours before the transfection, and the culture was continued. The medium was changed with 10 mL of fresh 10% FBS-containing DMEM after about 24 hours from the transfection, and then the culture was further continued. The cells were cultured at 37°C in a 5% CO₂ incubator. The culture solutions containing the viruses were collected after about 48 hours from the transfection, and through centrifugation and filtration with a filter, FVIII variant-producing lentiviral vectors were obtained as supernatants.

### (3) Acquisition of Human Preadipocytes for Introducing FVIII Variant Genes

Adipose tissue acquired from a healthy volunteer was dispensed in 50 mL centrifuge tubes each in an amount of 1 g, and after adding 3 mL of 2 mg/mL collagenase-containing Hank's balanced salt solution (Sigma-Aldrich Co. LLC), the tubes were shaken at 37°C for an hour. After an hour, after adding 10 mL of 20% FBS- and 40 µg/mL gentamicin-containing Dulbecco's Modified Eagle's Medium/Nutrient Mixture F-12 Ham (DMEM-HAM/20% FBS, Sigma-Aldrich Co. LLC, the same applies below) and stirring, the tubes were centrifuged at 400 g for a minute, and the solutions containing precipitate fractions were removed. Further, a series of operations of adding 10 mL of DMEM-HAM/20% FBS, stirring, then centrifuging at 400 g for a minute and removing the solutions containing precipitate fractions was conducted twice, and supernatant fractions containing preadipocytes were acquired. The supernatant fractions containing preadipocytes thus acquired were filtered with a 500 µm mesh into new 50 mL centrifuge tubes. Then, 10 mL of DMEM-HAM/20% FBS was added to the original 50 mL centrifuge tubes to wash the tubes, and the wash solutions were also filtered. Thus acquired filtrates were added to T150 flasks filled with DMEM-HAM/20% FBS which were warmed to 37°C in advance, and DMEM-HAM/20% FBS was further added in such a manner that air bubbles remaining in the flasks became as few as possible. Then, the flasks were placed in such a manner that the bottoms of the flasks became the culture surfaces (ceiling surfaces), and ceiling culture was conducted at 37°C in a 5% CO₂ incubator for seven days. After seven days, after removing the culture solutions in the flasks and washing the culture surfaces with PBS, the preadipocytes were collected by trypsin treatment, and the preadipocytes which were precipitated by centrifugation were suspended in a cell cryopreservation solution (CELLBANKER1: Takara Bio Inc., the same applies below) and frozen.

### (4) Production of FVIII Variant Gene-Introduced Preadipocytes

The preadipocytes frozen in (3) were thawed, suspended in MSF-BM/MSF-Supplement A (Shimadzu Diagnostics Corporation, the same applies below), precipitated by centrifugation to remove the cell cryopreservation solution, then suspended again in MSF-BM/MSF-Supplement A, put into a T225 flask and cultured using MSF-BM/MSF-Supplement A at 37°C in a 5% CO₂ incubator for four days. On the next day, after removing the culture solution in the flask and washing the culture surface with PBS, the preadipocytes were collected by trypsin treatment and precipitated by centrifugation. The preadipocytes which were precipitated by centrifugation were suspended in MSF-BM/MSF-Supplement A, and the cell concentration was measured. After 9.5×10⁵ cells were taken and precipitated by centrifugation, the cells were suspended in solutions obtained by mixing protamine sulfate to be a concentration of 100 µg/mL in the supernatants obtained in (2) (the FVIII variant-producing lentiviral vectors). Thus, cell suspensions having a final concentration of 4.75×10⁴ cells/mL were prepared. The cell suspensions after the transgenic operation in the above manner were seeded in 6-well plates at 2 mL/well, and sequential cell expansion was conducted while passaging the cells every three days or four days using MSF-BM/MSF-Supplement A at 37°C in a 5% CO₂ incubator from the next day, thereby, FVIII variant gene-introduced preadipocytes were produced.

### Experiment 2: Evaluation of Produced FVIII Variant Gene-Introduced Preadipocytes

The culture solutions in the flasks were removed during passaging of each of the five types of FVIII variant gene-introduced preadipocytes produced in Experiment 1 on the 10th to 14th day after seeding the cell suspensions in (4) in Experiment 1 in the 6-well plates, and the culture surfaces were washed with PBS. Then, a part of the cells collected by trypsin treatment were precipitated by centrifugation, suspended in a cell cryopreservation solution and frozen. The frozen cells were thawed, suspended in MSF-BM/MSF-Supplement A, precipitated by centrifugation to remove the cell cryopreservation solution, then suspended again in MSF-BM/MSF-Supplement A, put into T75 flasks and cultured using MSF-BM/MSF-Supplement A at 37°C in a 5% CO₂ incubator while passaging the cells every three days or four days. Passaging of the cells was conducted on the eighth day, the 11th day and the 14th day after starting the culture. The culture solutions in the flasks were removed during passaging on the 11th day and the 14th day, and the culture surfaces were washed with PBS. Then, the cells collected by trypsin treatment were precipitated by centrifugation and suspended in MSF-BM/MSF-Supplement A, and the cell concentrations were measured. After 2×10⁵ cells were taken and precipitated by centrifugation, the cells were suspended in PBS, frozen and stored until the cells were used for evaluation. Moreover, a part of the culture solutions removed from the flasks were also frozen and stored until the culture solutions were used for evaluation.

### (1) Calculation of Average Transgene Copy Numbers of FVIII Variant Genes

### (Calculation Method)

The frozen cells were thawed, and the DNA was extracted using DNeasy Blood & Tissue kit (QIAGEN N.V.) according to the protocol. Specifically, 20 µL of Proteinase K and 4 µL of RNase A (QIAGEN N.V.) were added to and mixed in the thawed cells, and after leaving still at room temperature for five minutes, 0.2 mL of Buffer AL was added and mixed. The mixtures were warmed at 56°C for 10 minutes. After 10 minutes, 0.2 mL of ethanol was added and mixed, and the mixtures were put in spin columns, set in 2 mL collection tubes, and the tubes were centrifuged at 6,000 g for a minute. The spin columns after centrifugation were set in new 2 mL collection tubes, and 0.5 mL of Buffer AW1 was added. Then, the tubes were centrifuged at 6,000 g for a minute. Next, the spin columns after centrifugation were set in new 2 mL collection tubes, and 0.5 mL of Buffer AW2 was added. Then the tubes were centrifuged at 20,000 g for three minutes. Next, the spin columns after centrifugation were set in new 1.5 mL collection tubes, and 0.2 mL of Buffer AE was added. After leaving still at room temperature for a minute, the tubes were centrifuged at 6,000 g for a minute to elute the DNA, and the concentrations were measured with an absorptiometer. From the DNA extracted from the cells in this manner, the average transgene copy numbers of the FVIII variant genes were calculated using QX200 Droplet Digital PCR System, C1000 Touch Thermal Cycler, PX1 PCR Plate Sealer and QuantaSoft Software of Bio-Rad Laboratories, Inc. according to the manual.

### (Calculation Results)

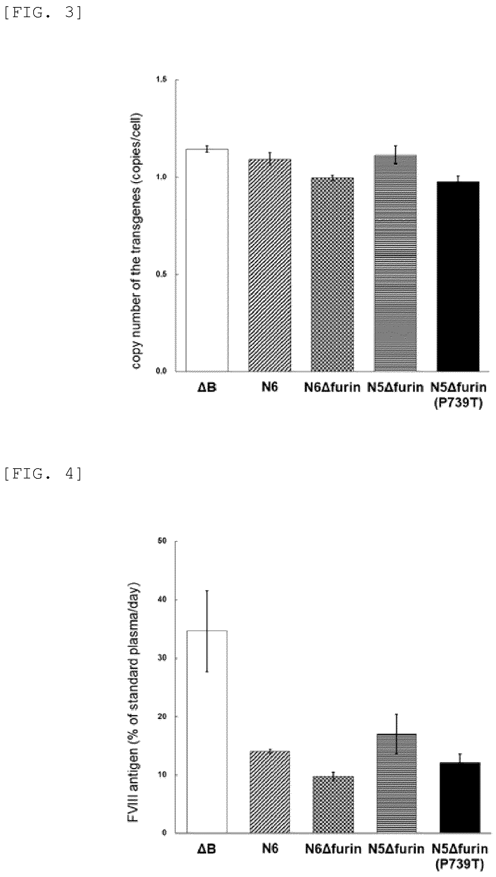

The average transgene copy numbers of each FVIII variant gene which were calculated from the DNA extracted from the cells collected during passaging on the 11th day and the 14th day after starting the culture in the T75 flask were added together and divided by 2, and the values thus obtained are shown in FIG. 3. As it is obvious from FIG. 3, in all the types of transgenic preadipocytes producing ΔB, N6, N6Δfurin, N5Δfurin and N5Δfurin (P739T), the average transgene copy numbers were similar, and there was no large difference.

### (2) Measurement of FVIII Antigen Amounts in Culture Solutions

The frozen culture solutions were thawed, and the FVIII antigen amounts in the culture solutions were measured using ELISA kit (Asserachrom FVIII, F. Hoffmann-La Roche, Ltd., the same applies below) according to the protocol. The FVIII antigen amounts were quantified based on the absorbances of a plate reader at 450 nm (subwavelength of 570 nm) as relative values (% values, where the FVIII antigen amount in the plasma of a healthy individual was regarded as 100%) based on the FVIII antigen amount in the plasma of a healthy individual from a created calibration curve of the FVIII antigen amount in the plasma of the healthy individual. The values obtained by dividing the FVIII antigen amounts in the culture solutions collected during passaging on the 11th day and the 14th day after starting the culture in the T75 flask by 3 (days of passaging) were added together and divided by 2, and the values thus obtained are shown in FIG. 4. As it is obvious from FIG. 4, the FVIII antigen amount in the culture solution of ΔB was a very high value, but the values of the other FVIII variants were a half or less of the value of ΔB, and there was no large difference.

### (3) Measurement of FVIII Activities of Culture Solutions

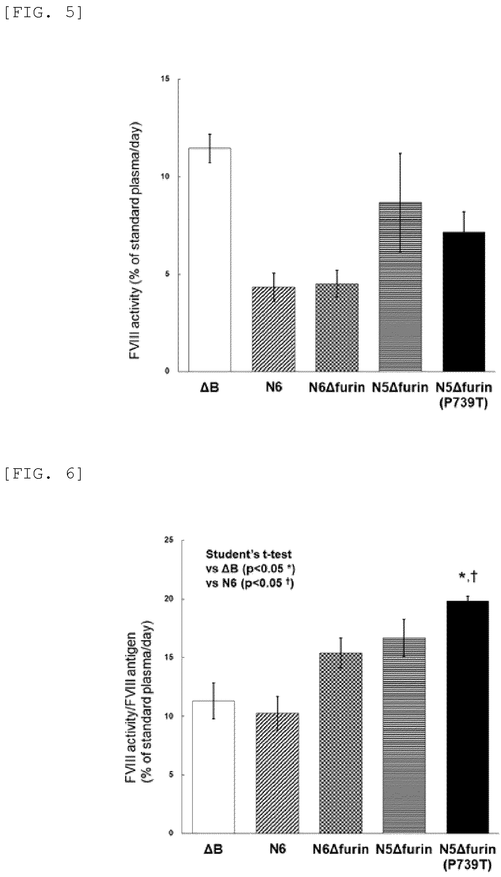

The frozen culture solutions were thawed, and the FVIII activities of the culture solutions were measured using Coatest SP FVIII kit (CHROMOGENIX AB) according to the protocol. The FVIII activities were quantified based on the absorbances of a plate reader at 450 nm (subwavelength of 570 nm) as relative values (% values, where the FVIII activity of the plasma of a healthy individual was regarded as 100%) based on the FVIII activity of the plasma of a healthy individual from a created calibration curve of the FVIII activity of the plasma of the healthy individual. The values obtained by dividing the FVIII activities of the culture solutions collected during passaging on the 11th day and the 14th day after starting the culture in the T75 flask by 3 (days of passaging) were added together and divided by 2, and the values thus obtained are shown in FIG. 5. As it is obvious from FIG. 5, the FVIII activity of the culture solution of ΔB was the highest value, and the value of N5Δfurin was the second highest. The value of N5Δfurin (P739T) was the next highest. The values of N6 and N6Δfurin were a half or less of the value of ΔB.

### (4) Calculation of Specific Activities (Activities per Antigen Amount) of FVIII Variants Secreted into Culture Solutions

From the results of (2) Measurement of FVIII Antigen Amounts in Culture Solutions and the results of (3) Measurement of FVIII Activities of Culture Solutions, the specific activities of the FVIII variants secreted into the culture solutions were calculated. The value obtained by dividing the FVIII activity of the culture solution collected during passaging on the 11th day after starting the culture in the T75 flask by 3 was divided by the value obtained by dividing the FVIII antigen amount in the culture solution by 3, and the value obtained by dividing the FVIII activity of the culture solution collected during passaging on the 14th day by 3 was divided by the value obtained by dividing the FVIII antigen amount in the culture solution by 3. The obtained values were added together and divided by 2. Thus obtained values are shown in FIG. 6. As it is obvious from FIG. 6, among the FVIII variants secreted into the culture solutions, N5Δfurin (P739T) showed the highest specific activity, and the value was far higher than the values of ΔB and N6 (with significant differences). The value of N5Δfurin was the second highest, and the value of N6Δfurin was the next highest. Both values were higher than the values of ΔB and N6 although there was no significant difference. Accordingly, it was found that the FVIII variants secreted from the transgenic preadipocytes producing N6Δfurin, N5Δfurin and N5Δfurin (P739T) into the culture solutions have high specific activities.

### Experiment 3: Transplantation of FVIII Variant Gene-Introduced Preadipocytes into NOD/SCID Mice

Each of the five types of FVIII variant gene-introduced preadipocytes produced in Experiment 1 was suspended in MSF-BM/MSF-Supplement A, seeded in any flask at 4×10⁴ cells/cm² and cultured using MSF-BM/MSF-Supplement A at 37°C in a 5% CO₂ incubator. On the next day, the medium was changed with PGM-2 (Lonza, the same applies below), and the cells were further cultured for three days. On the next day, after removing the culture solutions in the flasks and washing the culture surfaces with PBS, the cells were collected by trypsin treatment and precipitated by centrifugation. After washing the cells by conducting a series of operations of suspending the cells precipitated by centrifugation in Ringer's solution containing albumin at a final concentration of 0.5% and precipitating by centrifugation four times, an equivalent amount of Matrigel (Corning Incorporated, the same applies below) was mixed in 200 µg/mL trafermin-containing cell suspensions prepared by suspending the washed cells in Fiblast Spray (KAKEN PHARMACEUTICAL CO., LTD., the same applies below), and thus cell suspensions for transplantation having the final concentration of 2×10⁷ cells/mL (the final concentration of trafermin was 100 µg/mL) were prepared. The prepared cell suspensions for transplantation were administered subcutaneously into the backs on the left and the right of 7-week-old or 8-week-old NOD/SCID mice (The Jackson Laboratory Japan, Inc.) under isoflurane anesthesia using a 20G blunt cannula at 0.5 mL/site (2×10⁷ cells/animal).

### Experiment 4: Evaluation of FVIII Variant Gene-Introduced Preadipocytes as FVIII Variant-Producing Cells for ex vivo Gene Therapy (Measurement of FVIII Antigen Amounts in Blood of Transplanted Mice)

### (Measurement Method)

On the third day, the seventh day and the 14th day from the day on which the respective types of FVIII variant gene-introduced preadipocytes were transplanted into the NOD/SCID mice in Experiment 3, blood was collected from the cheeks of the mice under isoflurane anesthesia using Animal Lancet (MEDIpoint, Inc., the same applies below). The collected blood was put into 1.5 mL centrifuge tubes, and after adding EDTA to be a final concentration of 0.1 to 0.25% (v/v), plasma samples were acquired by centrifugation at 4°C at 5,000 rpm for 10 minutes, frozen and stored until the samples were used for evaluation. The frozen plasma samples were thawed, and the FVIII antigen amounts in the blood of the transplanted mice were measured using ELISA kit according to the protocol. The FVIII antigen amounts were quantified based on the absorbances of a plate reader at 450 nm (subwavelength of 570 nm) as relative values (% values, where the FVIII antigen amount in the plasma of a healthy individual was regarded as 100%) based on the FVIII antigen amount in the plasma of a healthy individual from a created calibration curve of the FVIII antigen amount in the plasma of the healthy individual.

### (Measurement Results)

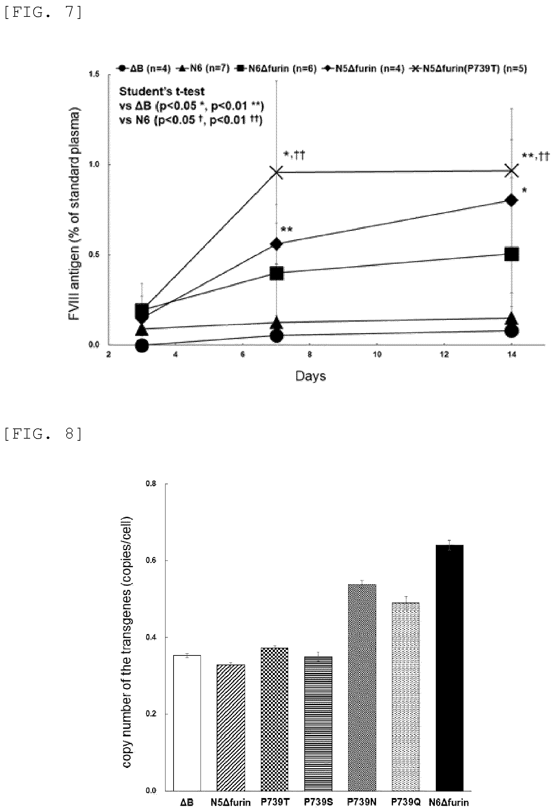

The measurement results are shown in FIG. 7. As it is obvious from FIG. 7, the FVIII antigen amount in the blood of the transplanted mouse of N5Δfurin (P739T) was the highest value and was far higher than the values of ΔB and N6 (with significant differences). The value of N5Δfurin was the second highest, and the value of N6Δfurin was the next highest. Both values were higher than the values of ΔB and N6 (the former had a significant difference from the value of ΔB). Accordingly, it was found that the secretion amounts of the FVIII variants into blood from the transgenic preadipocytes producing N6Δfurin, N5Δfurin and N5Δfurin (P739T) are higher than those from the transgenic preadipocytes producing ΔB and N6.

### Experiment B: Examination of Various FVIII Variant Gene-Introduced Preadipocytes (2)

### Experiment 1: Production of FVIII Variant Gene-Introduced Preadipocytes

### (1) Production of FVIII Variant-Producing Lentiviral Vector Plasmids

(f) An N5Δfurin (P739S)-producing lentiviral vector plasmid inserted a polynucleotide having a base sequence (SEQ ID NO: 17) corresponding to the amino acid sequence (SEQ ID NO: 14) of a protein in that a signal peptide is bonded to a FVIII variant (N5Δfurin (P739S)) in which the number of Asn residues subjected to N-linked glycosylation in the B domain of human FVIII is five from the N-terminus, which lacks the function of the furin recognition site and has substitution of the Pro residue at position 758 with a Ser residue into the SbfI/MluI site of pLVSIN-EF1α Neo Vector was produced by the following method. The N5Δfurin (P739T)-producing lentiviral vector plasmid produced in (e) in (1) in Experiment 1 of Experiment A was subjected to two PCR reactions using the two primer sets (Fw1&Re1 and Fw2&Re2) below, and two fragments, namely a fragment of a polynucleotide having the restriction enzyme site for XbaI at the 5' terminus and P739S mutation at the 3' terminus and a fragment of a polynucleotide having P739S mutation at the 5' terminus and the restriction enzyme site for MluI at the 3' terminus, were obtained using the polynucleotide having the base sequence corresponding to the amino acid sequence of the protein in which the signal peptide is bonded to N5Δfurin (P739T) inserted into the plasmid as a template. Thus obtained two polynucleotide fragments and a polynucleotide linearized by subjecting the N5Δfurin (P739T)-producing lentiviral vector plasmid to restriction enzyme treatment with XbaI and MluI were ligated by InFusion reaction to obtain the target N5Δfurin (P739S)-producing lentiviral vector plasmid.
   Fw1: GTGCGGCCCCTGTATTCTAG (SEQ ID NO: 20)
   Re1: CTTCTGCTCTCGATGGCATTG (SEQ ID NO: 21)
   Fw2: CATCGAGAGCAGAAGCTTCAGC (SEQ ID NO: 22)
   Re2: GTTGATTGTTCCAGACGCGTTC (SEQ ID NO: 23)
(g) An N5Δfurin (P739N)-producing lentiviral vector plasmid inserted a polynucleotide having a base sequence (SEQ ID NO: 18) corresponding to the amino acid sequence (SEQ ID NO: 15) of a protein in that the signal peptide is bonded to a FVIII variant (N5Δfurin (P739N)) in which the number of Asn residues subjected to N-linked glycosylation in the B domain of human FVIII is five from the N-terminus, which lacks the function of the furin recognition site and has substitution of the Pro residue at position 758 with an Asn residue into the SbfI/MluI site of pLVSIN-EF1α Neo Vector was produced by the same method as the method for producing the N5Δfurin (P739S)-producing lentiviral vector plasmid. The two primer sets (Fw1&Re1 and Fw2&Re2) which were used for subjecting the N5Δfurin (P739T)-producing lentiviral vector plasmid to two PCR reactions were as follows.
   Fw1: GTGCGGCCCCTGTATTCTAG (SEQ ID NO: 20)
   Re1: CTTCTGTTCTCGATGGCATTG (SEQ ID NO: 24)
   Fw2: CATCGAGAACAGAAGCTTCAGC (SEQ ID NO: 25)
   Re2: GTTGATTGTTCCAGACGCGTTC (SEQ ID NO: 23)
(h) An N5Δfurin (P739Q) -producing lentiviral vector plasmid inserted a polynucleotide having a base sequence (SEQ ID NO: 19) corresponding to the amino acid sequence (SEQ ID NO: 16) of a protein in that a signal peptide is bonded to a FVIII variant (N5Δfurin (P739Q)) in which the number of Asn residues subjected to N-linked glycosylation in the B domain of human FVIII is five from the N-terminus, which lacks the function of the furin recognition site and has substitution of the Pro residue at position 758 with a Gln residue into the SbfI/MluI site of pLVSIN-EF1α Neo Vector was produced by the same method as the method for producing the N5Δfurin (P739S)-producing lentiviral vector plasmid. The two primer sets (Fw1&Re1 and Fw2&Re2) which were used for subjecting the N5Δfurin (P739T)-producing lentiviral vector plasmid to two PCR reactions were as follows.
   Fw1: GTGCGGCCCCTGTATTCTAG (SEQ ID NO: 20)
   Re1: CTTCTCTGCTCGATGGCATTG (SEQ ID NO: 26)
   Fw2: CATCGAGCAGAGAAGCTTCAGC (SEQ ID NO: 27)
   Re2: GTTGATTGTTCCAGACGCGTTC (SEQ ID NO: 23)

### (2) Production of FVIII Variant-Producing Lentiviral Vectors

By the same method as the method for producing the FVIII variant-producing lentiviral vectors in (2) in Experiment 1 of Experiment A, FVIII variant-producing lentiviral vectors were produced using the FVIII variant-producing lentiviral vector plasmids produced in (1).

### (3) Acquisition of Human Preadipocytes for Introducing FVIII Variant Genes

By the same method as the method for acquiring human preadipocytes for introducing the FVIII variant genes in (3) in Experiment 1 of Experiment A, preadipocytes were acquired from adipose tissue acquired from a healthy volunteer, suspended in a cell cryopreservation solution and frozen.

### (4) Production of FVIII Variant Gene-Introduced Preadipocytes

The preadipocytes frozen in (3) were thawed, suspended in MSF-BM/MSF-Supplement A, precipitated by centrifugation to remove the cell cryopreservation solution, then suspended again in MSF-BM/MSF-Supplement A, put into a T150 flask and cultured using MSF-BM/MSF-Supplement A at 37°C in a 5% CO₂ incubator overnight. On the next day, after removing the culture solution in the flask and washing the culture surface with PBS, the preadipocytes were collected by trypsin treatment and precipitated by centrifugation. The preadipocytes which were precipitated by centrifugation were suspended in MSF-BM/MSF-Supplement A, and the cell concentration was measured. After 1.25×10⁵ cells were taken and precipitated by centrifugation, the cells were suspended in solutions obtained by mixing protamine sulfate to be a concentration of 100 µg/mL in the supernatants obtained in (2) (the FVIII variant-producing lentiviral vectors). Thus, cell suspensions having a final concentration of 2.5×10⁴ cells/mL were prepared. The cell suspensions after the transgenic operation in the above manner were seeded in T25 flasks at 5 mL/flask, and sequential cell expansion was conducted while passaging the cells every four days using MSF-BM/MSF-Supplement A at 37°C in a 5% CO₂ incubator from the next day. On the eighth day from the day on which the transgenic operation was conducted, the second transgenic operation was conducted in the same manner as above, and then, sequential cell expansion was conducted while passaging the cells every three days. Further, on the sixth day from the day on which the second transgenic operation was conducted (on the 14th day from the day on which the first transgenic operation was conducted), transgenic operation was conducted again in the same manner as above, and then, sequential cell expansion was conducted while passaging the cells every three days or four days. Thus, FVIII variant gene-introduced preadipocytes were produced (the number of transgenic operations was three in total).

### Experiment 2: Evaluation of Produced FVIII Variant Gene-Introduced Preadipocytes

Passaging of each of the three types of FVIII variant gene-introduced preadipocytes produced in Experiment 1 was conducted on the 25th day and the 29th day from the day on which the first transgenic operation was conducted in (4) in Experiment 1**.** The culture solutions in the flasks were removed during passaging on the 29th day, and the culture surfaces were washed with PBS. Then, the cells collected by trypsin treatment were precipitated by centrifugation and suspended in MSF-BM/MSF-Supplement **A,** and the cell concentrations were measured. After 2×10⁵ cells were taken and precipitated by centrifugation, the cells were suspended in PBS, frozen and stored until the cells were used for evaluation. Moreover, a part of the culture solutions removed from the flasks were also frozen and stored until the culture solutions were used for evaluation.

### (1) Calculation of Average Transgene Copy Numbers of FVIII Variant Genes

By the same method as the method for calculating the average transgene copy numbers of the FVIII variant genes in (1) in Experiment 2 of Experiment A, the average transgene copy numbers of each FVIII variant gene were calculated from the DNA extracted from the cells collected during passaging on the 29th day from the day on which the first transgenic operation was conducted. The results are shown in FIG. 8 (N5Δfurin (P739S), N5Δfurin (P739N) and N5Δfurin (P739Q) are described as P739S, P739N and P739Q, respectively, and the same applies below). Moreover, FIG. 8 also shows the results of the four types of FVIII variant gene-introduced preadipocytes which were produced by the same method as the method described in (2) to (4) in Experiment 1 using the ΔB-producing lentiviral vector plasmid, the N5Δfurin-producing lentiviral vector plasmid, the N5Δfurin (P739T)-producing lentiviral vector plasmid and the N6Δfurin-producing lentiviral vector plasmid, respectively, produced in (1) in Experiment 1 of Experiment A (N5Δfurin (P739T) is described as P739T, and the same applies below). As it is obvious from FIG. 8, in all the types of transgenic preadipocytes producing ΔB, N5Δfurin, N5Δfurin (P739T), N5Δfurin (P739S), N5Δfurin (P739N), N5Δfurin (P739Q) and N6Δfurin, the average transgene copy numbers were similar, and there was no large difference.

### (2) Measurement of FVIII Antigen Amounts in Culture Solutions

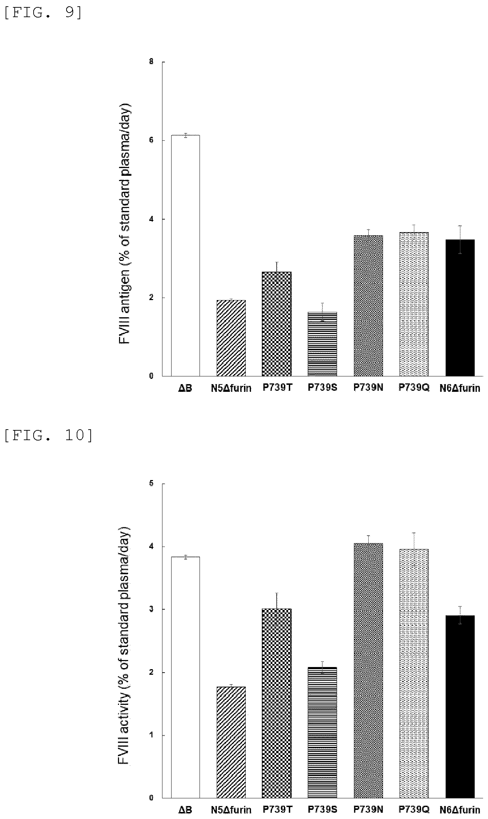

By the same method as the method for measuring the FVIII antigen amounts in the culture solutions in (2) in Experiment 2 of Experiment **A,** the FVIII antigen amounts in the culture solutions collected during passaging on the 29th day from the day on which the first transgenic operation was conducted were measured. The results are shown in FIG. 9. As it is obvious from FIG. 9, the FVIII antigen amount in the culture solution of ΔB was a very high value, but the values of the other FVIII variants were a half or less of the value of ΔB, and there was no large difference.

### (3) Measurement of FVIII Activities of Culture Solutions

The frozen culture solutions were thawed, and the FVIII activities of the culture solutions were measured using Revohem FVIII synthetic substrate kit (Sysmex Corporation) according to the protocol. The FVIII activities were quantified based on the absorbances of an automated blood coagulation analyzer (CS-1600, Sysmex Corporation) as relative values (% values, where the FVIII activity of the plasma of a healthy individual was regarded as 100%) based on the FVIII activity of the plasma of a healthy individual from a created calibration curve of the FVIII activity of the plasma of the healthy individual. The FVIII activities of the culture solutions collected during passaging on the 29th day from the day on which the first transgenic operation was conducted are shown in FIG. 10. As it is obvious from FIG. 10, the FVIII activities of the culture solutions of N5Δfurin (P739N) and N5Δfurin (P739Q) were high values that were equivalent to that of ΔB.

### (4) Calculation of Specific Activities (Activities per Antigen Amount) of FVIII Variants Secreted into Culture Solutions

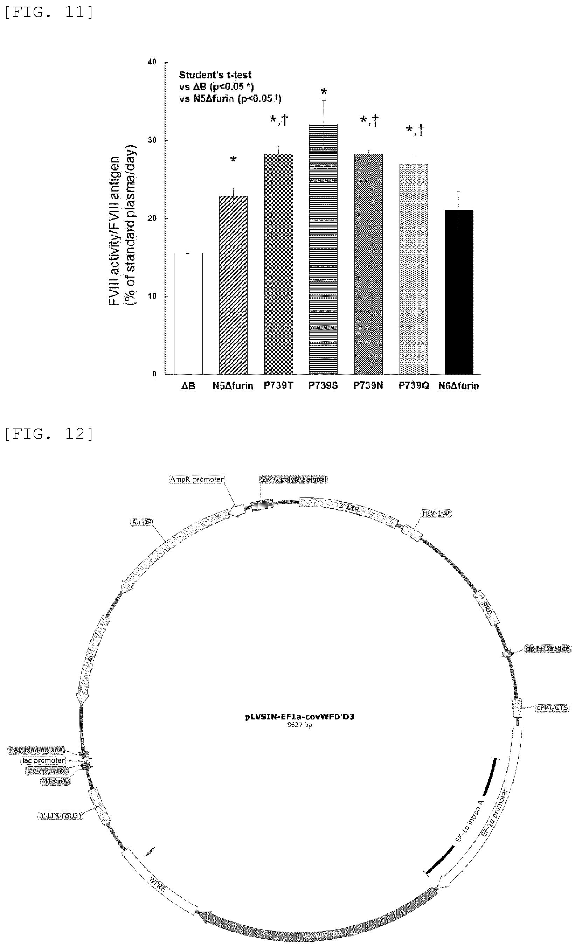

From the results of (2) Measurement of FVIII Antigen Amounts in Culture Solutions and the results of (3) Measurement of FVIII Activities of Culture Solutions, the specific activities of the FVIII variants secreted into the culture solutions were calculated. The results are shown in FIG. 11. As it is obvious from FIG. 11, among the FVIII variants secreted into the culture solutions, N5Δfurin (P739T), N5Δfurin (P739S), N5Δfurin (P739N) and N5Δfurin (P739Q) showed high specific activities, and the values were far higher than the value of ΔB (with significant differences). Moreover, the values were higher than the value of N5Δfurin (with significant differences except for N5Δfurin (P739S)). Accordingly, it was found that FVIII variants in which the Pro residue at position 758 of the amino acid sequence of N5Δfurin having the amino acid sequence at positions 20 to 1652 of the amino acid sequence of SEQ ID NO: 3 has been substituted with a polar uncharged amino acid residue such as Thr residue, Ser residue, Asn residue and Gln residue have high specific activities.

### Experiment C: Examination of Mixed Transplantation of FVIII Variant Gene-Introduced Preadipocytes and Human vWF Fragment Gene-Introduced Preadipocytes

### Experiment 1: Production of Human vWF Fragment Gene-Introduced Preadipocytes

### (1) Production of Human vWF Fragment-Producing Lentiviral Vector Plasmids

A polynucleotide in which the restriction enzyme sites for SbfI and MluI were added to the 5' terminus and the 3**'** terminus, respectively, of a base sequence (SEQ ID NO: 32) corresponding to the amino acid sequence (SEQ ID NO: 29) of a fragment (vWF1) composed of the SP, D1, D2, D' and D3 domains, the 26 amino acid residues from the N-terminus of the A1 domain, additional three amino acid residues and the CK domain of the domain structure SP-D1-D2-D'-D3-A1-A2-A3-D4-B1-B2-B3-C1-C2-CK which the precursor of human vWF has was synthesized and inserted into the SbfI/MluI site of pLVSIN-EF1α Neo Vector, and thus a vWF1-producing lentiviral vector plasmid was produced. Similarly, a vWF2-producing lentiviral vector plasmid in which a polynucleotide having a base sequence (SEQ ID NO: 33) corresponding to the amino acid sequence (SEQ ID NO: 30) of a fragment (vWF2) composed of the SP, D1, D2, D' and D3 domains and the 26 amino acid residues from the N-terminus of the A1 domain was inserted into the SbfI/MluI site of pLVSIN-EF1α Neo Vector, and a vWF3-producing lentiviral vector plasmid in which a polynucleotide having a base sequence (SEQ ID NO: 34) corresponding to the amino acid sequence (SEQ ID NO: 31) of a fragment (vWF3) composed of the SP, D**'** and D3 domains and the 26 amino acid residues from the N-terminus of the A1 domain was inserted into the SbfI/MluI site of pLVSIN-EF1α Neo Vector were produced. Here, as the base sequence of vWF1, the corresponding base sequence in the base sequence of human vWF (SEQ ID NO: 35) was used. Base sequences in which the codons were optimized were used for the base sequence of vWF2 and the base sequence of vWF3. The structure of the vWF3-producing lentiviral vector plasmid is shown in FIG. 12.

### (2) Production of Human vWF Fragment-Producing Lentiviral Vectors

By the same method as the method for producing the FVIII variant-producing lentiviral vectors in (2) in Experiment 1 of Experiment A, human vWF fragment-producing lentiviral vectors were produced using the human vWF fragment-producing lentiviral vector plasmids produced in (1).

### (3) Acquisition of Human Preadipocytes for Introducing Human vWF Fragment Genes

By the same method as the method for acquiring human preadipocytes for introducing the FVIII variant genes in (3) in Experiment 1 of Experiment A, preadipocytes were acquired from adipose tissue acquired from a healthy volunteer, suspended in a cell cryopreservation solution and frozen.

### (4) Production of Human vWF Fragment Gene-Introduced Preadipocytes

The preadipocytes frozen in (3) were thawed, suspended in MSF-BM/MSF-Supplement A, precipitated by centrifugation to remove the cell cryopreservation solution, then suspended again in MSF-BM/MSF-Supplement A, put into a T225 flask and cultured using MSF-BM/MSF-Supplement A at 37°C in a 5% CO₂ incubator for four days. On the next day, after removing the culture solution in the flask and washing the culture surface with PBS, the preadipocytes were collected by trypsin treatment and precipitated by centrifugation. The preadipocytes which were precipitated by centrifugation were suspended in MSF-BM/MSF-Supplement A, and the cell concentration was measured. After 2.5×10⁵ cells were taken and precipitated by centrifugation, the cells were suspended in solutions obtained by mixing protamine sulfate to be a concentration of 100 µg/mL in the supernatants obtained in (2) (the human vWF fragment-producing lentiviral vectors). Thus, cell suspensions having a final concentration of 5.0×10⁴ cells/mL were prepared. The cell suspensions after the transgenic operation in the above manner were seeded in T25 flasks at 5 mL/flask, and the cells were cultured using MSF-BM/MSF-Supplement A at 37°C in a 5% CO₂ incubator from the next day. On the second day from the day on which the transgenic operation was conducted, the second transgenic operation was conducted in the same manner as above, and then, sequential cell expansion was conducted while passaging the cells every three days. Further, on the sixth day from the day on which the second transgenic operation was conducted (on the eighth day from the day on which the first transgenic operation was conducted), transgenic operation was conducted again in the same manner as above, and then, sequential cell expansion was conducted while passaging the cells every three days or four days. Thus, vWF1 gene-introduced preadipocytes were produced. Moreover, on the second day from the day on which the transgenic operation was conducted, the second transgenic operation was conducted in the same manner as above, and then, the cells were cultured. Further, on the third day from the day on which the second transgenic operation was conducted (on the fifth day from the day on which the first transgenic operation was conducted), transgenic operation was conducted again in the same manner as above, and then, sequential cell expansion was conducted while passaging the cells every three days or four days. Thus, vWF2 gene-introduced preadipocytes and vWF3 gene-introduced preadipocytes were produced (the number of transgenic operations was three in total for the three types of transgenic preadipocytes).

### Experiment 2: Evaluation of Transplantation of Human vWF Fragment Gene-Introduced Preadipocytes as Mixture with FVIII Variant Gene-Introduced Preadipocytes (Measurement of FVIII Antigen Amounts in Blood of Transplanted Mice)

### (Measurement Method)

N5Δfurin (P739T) gene-introduced preadipocytes which were produced by the same method as the method described in (4) in Experiment 1 (here, the transgenic operations were conducted three times in total including those on the second day and the seventh day (on the fifth day from the day on which the second transgenic operation was conducted, without passaging in the period) from the day on which the first transgenic operation was conducted) using an N5Δfurin (P739T)-producing lentiviral vector produced by the same method as the method described in (2) in Experiment 1 of Experiment A using the N5Δfurin (P739T)-producing lentiviral vector plasmid produced in (1) in Experiment 1 of Experiment A and the human preadipocytes acquired in (3) in Experiment 1 and the three types of human vWF fragment gene-introduced preadipocytes produced in Experiment 1 were each suspended in MSF-BM/MSF-Supplement A, seeded in any flasks at 4×10⁴ cells/cm² and cultured using MSF-BM/MSF-Supplement A at 37°C in a 5% CO₂ incubator. On the next day, the medium was changed with PGM-2, and the cells were further cultured for three days. On the next day, after removing the culture solutions in the flasks and washing the culture surfaces with PBS, the cells were collected by trypsin treatment and precipitated by centrifugation. After washing the cells by conducting a series of operations of suspending the cells precipitated by centrifugation in Ringer's solution containing albumin at a final concentration of 0.5% and precipitating by centrifugation four times, the washed cells were suspended in a mixture solution of Fiblast Spray and MedGel (Nitta Gelatin Inc.), and thus 200 µg/mL trafermin- and 8 mg/mL MedGel-containing cell suspensions each having a cell concentration of 8×10⁷ cells/mL were prepared. Next, mixture suspensions of the N5Δfurin (P739T) gene-introduced preadipocytes and the human vWF fragment gene-introduced preadipocytes were prepared by mixing equivalent amounts of the prepared N5Δfurin (P739T) gene-introduced preadipocyte suspension and any of the human vWF fragment gene-introduced preadipocyte suspensions, and by further mixing an equivalent amount of Matrigel, cell suspensions for transplantation having the final concentrations of the respective cell types of 2×10⁷ cells/mL (the final concentration of trafermin was 100 µg/mL, and the final concentration of MedGel was 4 mg/mL) were prepared. The prepared cell suspensions for transplantation were administered subcutaneously into the backs on the left and the right of 6-week-old NOD/SCID mice (CLEA Japan, Inc., the same applies below) under isoflurane anesthesia using a 20G blunt cannula at 0.5 mL/site (2×10⁷ cells/animal for the two types of cells each). On the third, seventh, 14th, 21st and 28th days from the day on which the cell suspensions for transplantation containing the FVIII variant gene-introduced preadipocytes and the human vWF fragment gene-introduced preadipocytes were transplanted into the NOD/SCID mice, blood was collected from the cheeks of the mice under isoflurane anesthesia using Animal Lancet, and the FVIII antigen amounts in the blood of the transplanted mice were measured by the same method as the method for measuring the FVIII antigen amounts in the blood of the transplanted mice in Experiment 4 of Experiment A.

### (Measurement Results)

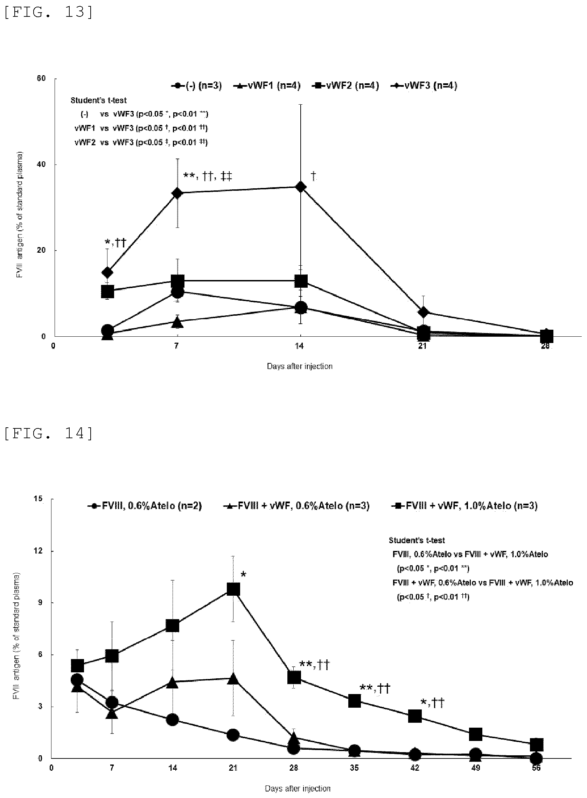

The measurement results are shown in FIG. 13. Moreover, a cell suspension for transplantation containing the N5Δfurin (P739T) gene-introduced preadipocytes alone at a final concentration of 2×10⁷ cells/mL was prepared and administered subcutaneously into the backs on the left and the right at 0.5 mL/site (2×10⁷ cells/animal) by the same methods as above, and the results are also shown in FIG. 13. As it is obvious from FIG. 13, the FVIII antigen amount in the blood of the transplanted mouse was higher when the N5Δfurin (P739T) gene-introduced preadipocytes were transplanted as a mixture with the vWF3 gene-introduced preadipocytes, compared to those of the case in which the N5Δfurin (P739T) gene-introduced preadipocytes alone were transplanted, the case in which the mixture with the vWF1 gene-introduced preadipocytes was transplanted and the case in which the mixture with the vWF2 gene-introduced preadipocytes was transplanted (with significant differences from the values of all the cases). Accordingly, it was found that the secretion amount of the FVIII variant into blood increases when human vWF fragment gene-introduced preadipocytes are transplanted as a mixture with FVIII variant gene-introduced preadipocytes.

### Experiment 3: Evaluation of Scaffold Material (Extracellular Matrix Component) for Transplantation of Human vWF Fragment Gene-Introduced Preadipocytes as Mixture with FVIII Variant Gene-Introduced Preadipocytes (Measurement of FVIII Antigen Amounts in Blood of Transplanted Mice)

### (Measurement Method)

The N5Δfurin (P739T) gene-introduced preadipocytes described in Experiment 2 and the vWF3 gene-introduced preadipocytes produced in Experiment 1 were each cultured, collected, precipitated by centrifugation, washed and then suspended in Ringer's solution containing albumin at a final concentration of 0.5% by the same method as the method described in Experiment 2, and thus cell suspensions having a cell concentration of 2×10⁸ cells/mL were prepared. Next, a mixture suspension of the N5Δfurin (P739T) gene-introduced preadipocytes and the vWF3 gene-introduced preadipocytes was prepared by mixing equivalent amounts of the prepared N5Δfurin (P739T) gene-introduced preadipocyte suspension and the vWF3 gene-introduced preadipocyte suspension, and by further mixing an equivalent amount of 1.2% or 2.0% atelocollagen (KOKEN CO., LTD., the same applies below), cell suspensions for transplantation having the final concentrations of the respective cell types of 5×10⁷ cells/mL (the final concentration of atelocollagen was 0.6% or 1.0%) were prepared. The prepared cell suspensions for transplantation were administered subcutaneously into the backs on the left and the right of 6-week-old NOD/SCID mice under isoflurane anesthesia using a 20G blunt cannula at 0.2 mL/site (2×10⁷ cells/animal for the two types of cells each). On the third, seventh, 14th, 21st, 28th, 35th, 42nd, 49th and 56th days from the day on which the cell suspensions for transplantation containing the FVIII variant gene-introduced preadipocytes and the vWF3 gene-introduced preadipocytes were transplanted into the NOD/SCID mice, blood was collected from the cheeks of the mice under isoflurane anesthesia using Animal Lancet, and the FVIII antigen amounts in the blood of the transplanted mice were measured by the same method as the method for measuring the FVIII antigen amounts in the blood of the transplanted mice in Experiment 4 of Experiment A.

### (Measurement Results)

The measurement results are shown in FIG. 14. Moreover, a cell suspension for transplantation containing the N5Δfurin (P739T) gene-introduced preadipocytes alone at a final concentration of 5×10⁷ cells/mL (the final concentration of atelocollagen was 0.6%) was prepared and administered subcutaneously into the backs on the left and the right at 0.2 mL/site (2×10⁷ cells/animal) by the same methods as above, and the results are also shown in FIG. 14. As it is obvious from FIG. 14, the FVIII antigen amount in the blood of the transplanted mouse was higher when atelocollagen was added to the cell suspensions for transplantation containing the N5Δfurin (P739T) gene-introduced preadipocytes and the vWF3 gene-introduced preadipocytes, compared to that of the case in which atelocollagen was added to the cell suspension for transplantation containing the N5Δfurin (P739T) gene-introduced preadipocytes alone, and the value of the case in which atelocollagen was added at a final concentration of 1.0% to the cell suspension for transplantation containing the N5Δfurin (P739T) gene-introduced preadipocytes and the vWF3 gene-introduced preadipocytes was higher for a long time than the values of the case in which atelocollagen was added at a final concentration of 0.6% to the cell suspension for transplantation containing the N5Δfurin (P739T) gene-introduced preadipocytes alone and the case in which atelocollagen was added at a final concentration of 0.6% to the cell suspension for transplantation containing the N5Δfurin (P739T) gene-introduced preadipocytes and the vWF3 gene-introduced preadipocytes (with significant differences from the values of both cases). Accordingly, it was found that the secretion amount of the FVIII variant into blood increases when atelocollagen is used as a scaffold material for transplantation of human vWF fragment gene-introduced preadipocytes as a mixture with FVIII variant gene-introduced preadipocytes.

### Experiment 4: Evaluation of Transplant Site for Transplantation of Human vWF Fragment Gene-Introduced Preadipocytes as Mixture with FVIII Variant Gene-Introduced Preadipocytes (Measurement of FVIII Antigen Amounts in Blood of Transplanted Mice)

### (Measurement Method)

The N5Δfurin (P739T) gene-introduced preadipocytes described in Experiment 2 and the vWF3 gene-introduced preadipocytes produced in Experiment 1 were each cultured, collected, precipitated by centrifugation, washed and then suspended in Ringer's solution containing albumin at a final concentration of 0.5% by the same method as the method described in Experiment 2, and thus cell suspensions having a cell concentration of 2×10⁸ cells/mL were prepared. Next, a mixture suspension of the N5Δfurin (P739T) gene-introduced preadipocytes and the vWF3 gene-introduced preadipocytes was prepared by mixing equivalent amounts of the prepared N5Δfurin (P739T) gene-introduced preadipocyte suspension and the vWF3 gene-introduced preadipocyte suspension, and by further mixing an equivalent amount of 2.0% atelocollagen, a cell suspension for transplantation having the final concentrations of the respective cell types of 5×10⁷ cells/mL (the final concentration of atelocollagen was 1.0%) was prepared. The prepared cell suspension for transplantation was administered subcutaneously into the backs on the left and the right of 6-week-old NOD/SCID mice under isoflurane anesthesia using a 20G blunt cannula or into the inguinals on the left and the right using a 25G injection needle at 0.2 mL/site (2×10⁷ cells/animal for the two types of cells each). On the third, seventh, 14th, 21st, 28th, 35th, 42nd, 49th and 56th days from the day on which the cell suspension for transplantation containing the FVIII variant gene-introduced preadipocytes and the vWF3 gene-introduced preadipocytes was transplanted into the NOD/SCID mice, blood was collected from the cheeks of the mice under isoflurane anesthesia using Animal Lancet, and the FVIII antigen amounts in the blood of the transplanted mice were measured by the same method as the method for measuring the FVIII antigen amounts in the blood of the transplanted mice in Experiment 4 of Experiment A.

### (Measurement Results)

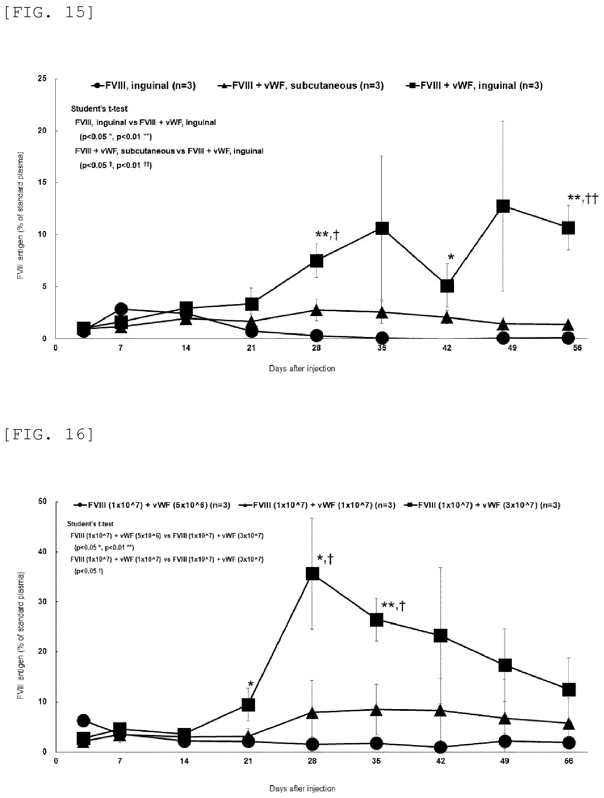

The measurement results are shown in FIG. 15. Moreover, a cell suspension for transplantation containing the N5Δfurin (P739T) gene-introduced preadipocytes alone at a final concentration of 5×10⁷ cells/mL (the final concentration of atelocollagen was 1.0%) was prepared and administered into the inguinals on the left and the right at 0.2 mL/site (2×10⁷ cells/animal) by the same methods as above, and the results are also shown in FIG. 15. As it is obvious from FIG. 15, the FVIII antigen amount in the blood of the transplanted mouse was higher when the mixture of the N5Δfurin (P739T) gene-introduced preadipocytes and the vWF3 gene-introduced preadipocytes was transplanted inguinally, compared to those of the case in which the N5Δfurin (P739T) gene-introduced preadipocytes alone were transplanted inguinally and the case in which the mixture of the N5Δfurin(P739T) gene-introduced preadipocytes and the vWF3 gene-introduced preadipocytes was subcutaneously transplanted into the back (with significant differences from the values of both cases). Accordingly, it was found that the secretion amount of the FVIII variant into blood increases when the transplant site for transplantation of human vWF fragment gene-introduced preadipocytes as a mixture with FVIII variant gene-introduced preadipocytes is the inguinals.

### Experiment 5: Evaluation of Ratio of Transplanted Cell Counts for Transplantation of Human vWF Fragment Gene-Introduced Preadipocytes as Mixture with FVIII Variant Gene-Introduced Preadipocytes (Measurement of FVIII Antigen Amounts in Blood of Transplanted Mice)

### (Measurement Method)

The N5Δfurin (P739T) gene-introduced preadipocytes described in Experiment 2 and the vWF3 gene-introduced preadipocytes produced in Experiment 1 were each cultured, collected, precipitated by centrifugation, washed and then suspended in Ringer's solution containing albumin at a final concentration of 0.5% by the same method as the method described in Experiment 2, and thus cell suspensions having the cell concentrations described below were prepared.
(N5Δfurin (P739T) gene-introduced preadipocyte suspensions)
   · suspension of 5×10⁷ cells/mL
   · suspension of 3.3×10⁷ cells/mL
(vWF3 gene-introduced preadipocyte suspensions)
   · suspension of 1×10⁸ cells/mL
   · suspension of 5×10⁷ cells/mL
   · suspension of 2.5×10⁷ cells/mL

Next, mixture suspensions of the N5Δfurin (P739T) gene-introduced preadipocytes and the vWF3 gene-introduced preadipocytes were prepared by mixing equivalent amounts of any of the prepared N5Δfurin (P739T) gene-introduced preadipocyte suspensions and any of the prepared vWF3 gene-introduced preadipocyte suspensions, and by further mixing an equivalent amount of 1.6% atelocollagen, the three types of cell suspension for transplantation having the final concentrations of the respective cell types described below (the final concentration of atelocollagen was 0.8%) were prepared.
(1) The N5Δfurin (P739T) gene-introduced preadipocytes were at 1.25×10⁷ cells/mL and the vWF3 gene-introduced preadipocytes were at 6.25×10⁶ cells/mL.
(2) The N5Δfurin (P739T) gene-introduced preadipocytes were at 1.25×10⁷ cells/mL and the vWF3 gene-introduced preadipocytes were at 1.25×10⁷ cells/mL.
(3) The N5Δfurin (P739T) gene-introduced preadipocytes were at 8.25×10⁶ cells/mL and the vWF3 gene-introduced preadipocytes were at 2.5×10⁷ cells/mL.

The prepared three types of cell suspension for transplantation were each administered into the inguinals on the left and the right of 6-week-old NOD/SCID mice under isoflurane anesthesia using a 25G injection needle, at 0.4 mL/site for the cell suspension (1) for transplantation (N5Δfurin (P739T) gene-introduced preadipocytes were at 1×10⁷ cells/animal and the vWF3 gene-introduced preadipocytes were at 5×10⁶ cells/animal), also at 0.4 mL/site for the cell suspension (2) for transplantation (1×10⁷ cells/animal for the two types of cells each) and at 0.6 mL/site for the cell suspension (3) for transplantation (N5Δfurin (P739T) gene-introduced preadipocytes were at 1×10⁷ cells/animal and the vWF3 gene-introduced preadipocytes were at 3×10⁷ cells/animal). On the third, seventh, 14th, 21st, 28th, 35th, 42nd, 49th and 56th days from the day on which the cell suspensions for transplantation containing the FVIII variant gene-introduced preadipocytes and the vWF3 gene-introduced preadipocytes were transplanted into the NOD/SCID mice, blood was collected from the cheeks of the mice under isoflurane anesthesia using Animal Lancet, and the FVIII antigen amounts in the blood of the transplanted mice were measured by the same method as the method for measuring the FVIII antigen amounts in the blood of the transplanted mice in Experiment 4 of Experiment A.

### (Measurement Results)

The measurement results are shown in FIG. 16. As it is obvious from FIG. 16, the FVIII antigen amount in the blood of the transplanted mouse was higher as the ratio of the transplanted cell count of the vWF3 gene-introduced preadipocytes to the transplanted cell count of the N5Δfurin (P739T) gene-introduced preadipocytes became higher, and the value of the case in which the cell suspension (3) for transplantation was administered was higher than the values of the case in which the cell suspension (1) for transplantation was administered and the case in which the cell suspension (2) for transplantation was administered (with significant differences from the values of both cases). Accordingly, it was found that the secretion amount of the FVIII variant into blood increases when the ratio of the transplanted cell count of the human vWF fragment to the transplanted cell count of the FVIII variant gene-introduced preadipocytes is set high for transplantation of human vWF fragment gene-introduced preadipocytes as a mixture with FVIII variant gene-introduced preadipocytes.

### Industrial Applicability

The present invention is industrially applicable because the present invention can provide a novel cell for *ex vivo* gene therapy which produces a FVIII variant.

## Claims

1. A cell for *ex vivo* gene therapy which produces a variant of human FVIII having the amino acid sequence at positions 20 to 2351 of the amino acid sequence of SEQ ID NO: 1, wherein in the FVIII variant, the B domain (the amino acid sequence at positions 760 to 1667) has been modified, thereby, the number of Asn residues subjected to N-linked glycosylation is four to six from the N-terminus and the function of the furin recognition site is lacking.

2. The cell for *ex vivo* gene therapy according to claim 1, wherein the FVIII variant is any of (a) to (c) below:
(a) a protein having the amino acid sequence at positions 20 to 1607 of the amino acid sequence of SEQ ID NO: 2;
(b) a protein which has an amino acid sequence containing deletion, substitution, addition or insertion of one or a plurality of amino acid residues in the amino acid sequence at positions 20 to 1607 of the amino acid sequence of SEQ ID NO: 2 and which has an equivalent biological activity to that of the protein in (a); and
(c) a protein which has an amino acid sequence having a sequence identity of at least 90% with the amino acid sequence at positions 20 to 1607 of the amino acid sequence of SEQ ID NO: 2 and which has an equivalent biological activity to that of the protein in (a).

3. The cell for *ex vivo* gene therapy according to claim 1, wherein the FVIII variant is any of (a) to (c) below:
(a) a protein having the amino acid sequence at positions 20 to 1652 of the amino acid sequence of SEQ ID NO: 3;
(b) a protein which has an amino acid sequence containing deletion, substitution, addition or insertion of one or a plurality of amino acid residues in the amino acid sequence at positions 20 to 1652 of the amino acid sequence of SEQ ID NO: 3 and which has an equivalent biological activity to that of the protein in (a); and
(c) a protein which has an amino acid sequence having a sequence identity of at least 90% with the amino acid sequence at positions 20 to 1652 of the amino acid sequence of SEQ ID NO: 3 and which has an equivalent biological activity to that of the protein in (a).

4. The cell for *ex vivo* gene therapy according to claim 1, wherein the FVIII variant is any of (a) to (c) below:
(a) a protein having the amino acid sequence at positions 20 to 1670 of the amino acid sequence of SEQ ID NO: 4;
(b) a protein which has an amino acid sequence containing deletion, substitution, addition or insertion of one or a plurality of amino acid residues in the amino acid sequence at positions 20 to 1670 of the amino acid sequence of SEQ ID NO: 4 and which has an equivalent biological activity to that of the protein in (a); and
(c) a protein which has an amino acid sequence having a sequence identity of at least 90% with the amino acid sequence at positions 20 to 1670 of the amino acid sequence of SEQ ID NO: 4 and which has an equivalent biological activity to that of the protein in (a).

5. The cell for *ex vivo* gene therapy according to claim 1, wherein in the FVIII variant, the Pro residue at position 758 of the amino acid sequence of SEQ ID NO: 1 has been substituted with another amino acid residue.

6. The cell for *ex vivo* gene therapy according to claim 5, wherein the other amino acid residue is a polar uncharged amino acid residue selected from Thr residue, Ser residue, Asn residue and Gln residue.

7. The cell for *ex vivo* gene therapy according to claim 5, wherein the FVIII variant is any of (a) to (c) below:
(a) a protein having the amino acid sequence at positions 20 to 1652 of the amino acid sequence of SEQ ID NO: 5;
(b) a protein which has an amino acid sequence containing deletion, substitution, addition or insertion of one or a plurality of amino acid residues in the amino acid sequence at positions 20 to 1652 of the amino acid sequence of SEQ ID NO: 5 and which has an equivalent biological activity to that of the protein in (a); and
(c) a protein which has an amino acid sequence having a sequence identity of at least 90% with the amino acid sequence at positions 20 to 1652 of the amino acid sequence of SEQ ID NO: 5 and which has an equivalent biological activity to that of the protein in (a).

8. The cell for *ex vivo* gene therapy according to claim 5, wherein the FVIII variant is any of (a) to (c) below:
(a) a protein having the amino acid sequence at positions 20 to 1652 of the amino acid sequence of SEQ ID NO: 14;
(b) a protein which has an amino acid sequence containing deletion, substitution, addition or insertion of one or a plurality of amino acid residues in the amino acid sequence at positions 20 to 1652 of the amino acid sequence of SEQ ID NO: 14 and which has an equivalent biological activity to that of the protein in (a); and
(c) a protein which has an amino acid sequence having a sequence identity of at least 90% with the amino acid sequence at positions 20 to 1652 of the amino acid sequence of SEQ ID NO: 14 and which has an equivalent biological activity to that of the protein in (a).

9. The cell for *ex vivo* gene therapy according to claim 5, wherein the FVIII variant is any of (a) to (c) below:
(a) a protein having the amino acid sequence at positions 20 to 1652 of the amino acid sequence of SEQ ID NO: 15;
(b) a protein which has an amino acid sequence containing deletion, substitution, addition or insertion of one or a plurality of amino acid residues in the amino acid sequence at positions 20 to 1652 of the amino acid sequence of SEQ ID NO: 15 and which has an equivalent biological activity to that of the protein in (a); and
(c) a protein which has an amino acid sequence having a sequence identity of at least 90% with the amino acid sequence at positions 20 to 1652 of the amino acid sequence of SEQ ID NO: 15 and which has an equivalent biological activity to that of the protein in (a).

10. The cell for *ex vivo* gene therapy according to claim 5, wherein the FVIII variant is any of (a) to (c) below:
(a) a protein having the amino acid sequence at positions 20 to 1652 of the amino acid sequence of SEQ ID NO: 16;
(b) a protein which has an amino acid sequence containing deletion, substitution, addition or insertion of one or a plurality of amino acid residues in the amino acid sequence at positions 20 to 1652 of the amino acid sequence of SEQ ID NO: 16 and which has an equivalent biological activity to that of the protein in (a); and
(c) a protein which has an amino acid sequence having a sequence identity of at least 90% with the amino acid sequence at positions 20 to 1652 of the amino acid sequence of SEQ ID NO: 16 and which has an equivalent biological activity to that of the protein in (a).

11. The cell for *ex vivo* gene therapy according to claim **1,** wherein the cell is an adipocyte.

12. A composition for transplantation comprising the cell for *ex vivo* gene therapy according to claim 1 and a pharmaceutically acceptable carrier.

13. The composition for transplantation according to claim 12 which further comprises an extracellular matrix component.

14. The composition for transplantation according to claim 12 which further comprises an angiogenic factor.

15. A method for producing a cell for *ex vivo* gene therapy comprising introducing an expression vector having a polynucleotide which encodes at least an amino acid sequence of a variant of human FVIII having the amino acid sequence at positions 20 to 2351 of the amino acid sequence of SEQ ID NO: 1, wherein in the FVIII variant, the B domain (the amino acid sequence at positions 760 to 1667) has been modified, thereby, the number of Asn residues subjected to N-linked glycosylation is four to six from the N-terminus and the function of the furin recognition site is lacking, into a cell which can be transplanted into the body of a subject of the *ex vivo* gene therapy.

16. The method for producing a cell for *ex vivo* gene therapy according to claim 15, wherein the expression vector is a viral vector.

17. The method for producing a cell for *ex vivo* gene therapy according to claim 16, wherein the viral vector is a lentiviral vector.

18. An expression vector for producing a cell for *ex vivo* gene therapy comprising a polynucleotide which encodes at least an amino acid sequence of a variant of human FVIII having the amino acid sequence at positions 20 to 2351 of the amino acid sequence of SEQ ID NO: 1, wherein in the FVIII variant, the B domain (the amino acid sequence at positions 760 to 1667) has been modified, thereby, the number of Asn residues subjected to N-linked glycosylation is four to six from the N-terminus and the function of the furin recognition site is lacking.

19. A variant of human FVIII having the amino acid sequence at positions 20 to 2351 of the amino acid sequence of SEQ ID NO: 1, wherein in the FVIII variant, the B domain (the amino acid sequence at positions 760 to 1667) has been modified, thereby, the number of Asn residues subjected to N-linked glycosylation is four to six from the N-terminus, the function of the furin recognition site is lacking and the Pro residue at position 758 has been substituted with another amino acid residue.

20. The FVIII variant according to claim 19, wherein the other amino acid residue is a polar uncharged amino acid residue selected from Thr residue, Ser residue, Asn residue and Gln residue.

21. A polynucleotide which encodes at least an amino acid sequence of the FVIII variant according to claim 19 or 20.

22. The composition for transplantation according to any of claims 12 to 14 which further comprises a cell that produces human vWF and/or a fragment thereof *in vivo.*

23. A cell which produces a fragment of human vWF *in vivo.*

24. The cell according to claim 23, wherein the fragment of human vWF contains at least the D' domain and the D3 domain, which form a binding site for FVIII.

25. The cell according to claim 23, wherein the fragment of human vWF is a fragment which has the SP, D' and D3 domains, does not have any of the A2, A3, D4, B1, B2, B3, C1 and C2 domains, may have 50 amino acid residues maximum from the N-terminus of the A1 domain and may have but does not have to have the D1, D2 and CK domains of a domain structure SP-D1-D2-D'-D3-A1-A2-A3-D4-B1-B2-B3-C1-C2-CK which a precursor has.

26. The cell according to claim 24, wherein the fragment of human vWF is any of (a) to (c) below:
(a) a protein having the amino acid sequence of SEQ ID NO: 29;
(b) a protein which has an amino acid sequence containing deletion, substitution, addition or insertion of one or a plurality of amino acid residues in the amino acid sequence of SEQ ID NO: 29 and which has an equivalent biological activity to that of the protein in (a); and
(c) a protein which has an amino acid sequence having a sequence identity of at least 90% with the amino acid sequence of SEQ ID NO: 29 and which has an equivalent biological activity to that of the protein in (a).

27. The cell according to claim 24, wherein the fragment of human vWF is any of (a) to (c) below:
(a) a protein having the amino acid sequence of SEQ ID NO: 30;
(b) a protein which has an amino acid sequence containing deletion, substitution, addition or insertion of one or a plurality of amino acid residues in the amino acid sequence of SEQ ID NO: 30 and which has an equivalent biological activity to that of the protein in (a); and
(c) a protein which has an amino acid sequence having a sequence identity of at least 90% with the amino acid sequence of SEQ ID NO: 30 and which has an equivalent biological activity to that of the protein in (a).

28. The cell according to claim 24, wherein the fragment of human vWF is any of (a) to (c) below:
(a) a protein having the amino acid sequence of SEQ ID NO: 31;
(b) a protein which has an amino acid sequence containing deletion, substitution, addition or insertion of one or a plurality of amino acid residues in the amino acid sequence of SEQ ID NO: 31 and which has an equivalent biological activity to that of the protein in (a); and
(c) a protein which has an amino acid sequence having a sequence identity of at least 90% with the amino acid sequence of SEQ ID NO: 31 and which has an equivalent biological activity to that of the protein in (a).

29. A drug which is for increasing the secretion amount of the FVIII variant produced by transplanting the cell for *ex vivo* gene therapy according to claim 1 into the body of a subject of the *ex vivo* gene therapy into blood and which comprises a cell that produces human vWF and/or a fragment thereof *in vivo* as an active ingredient.

30. A composition for transplantation which comprises a cell that produces human vWF and/or a fragment thereof *in vivo* and a pharmaceutically acceptable carrier.

31. A set of compositions for transplantation which comprises the composition for transplantation according to claim 12 and the composition for transplantation according to claim 30.
